# EUROPEAN PATENT APPLICATION

(11) **EP 4 431 522 A1**
(43) Date of publication of application: **18.09.2024**
(21) Application number: 22892094.8
(22) Date of filing: 11.11.2022
(51) Int. Cl.: C07K 14/605, A61K 38/26, A61P 3/10, A61P 3/04

(54) **PHARMACEUTICAL COMPOSITION OF GLP-1 RECEPTOR AND GIP RECEPTOR DUAL AGONIST, AND USE THEREOF**

(30) Priority: 12.11.2021 CN 202111341752
(71) Applicant: Fujian Shengdi Pharmaceutical Co., Ltd., Xiamen, Fujian 361026 (CN); Jiangsu Hengrui Pharmaceuticals Co., Ltd., Lianyungang, Jiangsu 222047 (CN)
(72) Inventor: LI, Zhenbin, Xiamen, Fujian 361026 (CN); CHEN, Jing, Xiamen, Fujian 361026 (CN); CAO, Xueteng, Xiamen, Fujian 361026 (CN); LIU, Kai, Xiamen, Fujian 361026 (CN)
(74) Representative: Potter Clarkson
(86) International application number: PCT/CN2022/131375
(87) International publication number: WO 2023/083301

(57) **Abstract**

Provided are a pharmaceutical composition of a GLP-1 receptor and a GIP receptor dual agonist, and a use thereof. Specifically, the pharmaceutical composition comprises a GLP-1 analogue as shown in general formula (I), and a buffer such as a phosphate buffer; the composition may further comprise an osmoregulator such as propylene glycol, sodium chloride or mannitol. The pharmaceutical composition has good biological activity and stability. The general formula (I) is:

R1-X1-X2-Glu-Gly-Thr-Phe-Thr-Ser-Asp-X10-Ser-X12-X13-X14-X15-X16-X17-X18-X19-X20
-Glu-Phe-X23-X24-Trp-Leu-X27-X28-X29-X30-Pro-Ser-Ser-Gly-Ala-Pro-Pro-Pro-Ser-R2.
(I)

## Description

### TECHNICAL FIELD

The present disclosure relates to the field of pharmaceutical formulations, in particular to a pharmaceutical composition comprising a GLP-1 receptor and GIP receptor dual-agonist and pharmaceutical use thereof.

### BACKGROUND

At present, many GLP-1 drugs are available on the market and are mainly used for treating T2DM, such as liraglutide, semaglutide, and dulaglutide, where liraglutide is also approved for marketing by FDA as a weight-loss drug. Under physiological conditions, active GLP-1 consists of 30 amino acids and is secreted by cleavage of pro-glucagon by the PC1/3 enzyme in L cells of the intestinal tract after meals. In a T2DM patient, the secretion of GLP-1 after meals is significantly inhibited, but the response of GLP-1R of the patient to GLP-1 at a pharmacological concentration is not significantly different from that of a normal person, which further proves that the target is of great treatment potential. Although GLP-1R agonists exhibit great advantages in terms of the therapeutic effects such as blood glucose lowering and weight loss, their actions on the central nervous system and stomach would cause nausea and vomiting, that is, there would be dose-dependent gastrointestinal adverse reactions. If the therapeutic dose of the GLP-1 drugs is limited and one can not continue to increase the dose to achieve more remarkable treatment effects in glucose lowering, weight loss, etc., there is a need to supplement with other treatment schemes to enhance the treatment effect or reduce the occurrence rate of adverse reactions of the GLP-1 drugs.

Glucose-dependent insulinotropic polypeptide (GIP) also belongs to the incretins. The active GIP contains 42 amino acids and is produced by the cleavage of the GIP precursor by the PC1/3 enzyme in K cells secreted in the intestines. The GIP can play a role in comprehensively regulating the nervous system and the endocrine system at the same time and thereby improve metabolism. GLP-1 can be effective by directly influencing the central nervous system, pancreas and stomach and indirectly acting on liver, plus the active ingredient taking effect on fat and muscle tissues, thereby comprehensively improving metabolism. Studies have shown that in non-insulin-dependent diabetic patients, the incretin function of GIP polypeptide is greatly reduced, resulting in a lack or loss of incretin effect in the patients. Studies have shown that the inhibitory properties of the GIP polypeptide produced by those diabetic patients are greatly diminished when the blood glucose level returns to normal.

Therefore, there is a clinical need for a method for treating non-insulin-dependent diabetes using the GIP polypeptide in combination with a clinically effective drug for lowering blood glucose to restore the tolerance of the non-insulin-dependent diabetic patients to the GIP polypeptide, and further in combination with the incretin effect of the GIP polypeptide to obtain a stronger clinical glucose lowering effect.

PCT/CN2021/096568 provides a derivative of a GLP-1 analog having agonist activity for human GIP receptor, which has dual-agonist effect on human GLP-1 receptor and human GIP receptor; compared with GLP-1 receptor agonists known in the art, the derivative has stronger curative effects of glucose lowering and weight loss, has extremely high plasma stability, and has the pharmacokinetic characteristics of once-a-week subcutaneous injection on human subjects. However, chemically modified polypeptide drugs have a complex structure, are easily degraded, polymerized, or undesirably chemically modified, and thus become unstable; in order to make them suitable for administration, maintain stability during storage and subsequent use, and exert a better therapeutic effect, it is particularly important to develop stable formulations of the chemically modified polypeptide drugs.

### SUMMARY

The present disclosure provides a pharmaceutical composition, which comprises:
(a) a GLP-1 analog of general formula (I) or a pharmaceutically acceptable salt thereof 
R1-X1-X2-Glu-Gly-Thr-Phe-Thr-Ser-Asp-X10-Ser-X12-X13-X14-X15-X16-X17-X18-X19-X20
-Glu-Phe-X23-X24-Trp-Leu-X27-X28-X29-X30-Pro-Ser-Ser-Gly-Ala-Pro-Pro-Pro-Ser-R2
(I) (SEQ ID NO: 19)
 wherein:
R₁ is hydrogen (H), alkyl, acetyl, formyl, benzoyl, trifluoroacetyl, pGlu, or absent;
R₂ is -NH₂, -OH, or absent;
X₁, X₂, X₁₀, X₁₂, X₁₃, X₁₄, X₁₅, X₁₆, X₁₇, X₁₈, X₁₉, X₂₀, X₂₃, X₂₄, X₂₇, X₂₈, X₂₉, and X₃₀ are independently selected from the group consisting of any natural amino acid residues, any non-natural amino acid residues, and peptide fragments composed of natural amino acid residues and/or non-natural amino acid residues; and
at least one selected from the group consisting of (b) a buffer, (c) an osmotic pressure regulator, (d) a pH adjuster, and (e) a bacteriostatic agent.

In some embodiments, the pharmaceutical composition comprises the GLP-1 analog of general formula (I) or the pharmaceutically acceptable salt thereof and a buffer.

In some embodiments, the buffer is selected from one or more of an acetate buffer, a histidine salt buffer, a phosphate buffer, a succinate buffer, and a citric acid buffer.

In some embodiments, the buffer is a phosphate buffer, such as disodium hydrogen phosphate.

In some embodiments, X₁ is Tyr or His; X₂ is Aib or D-Ala; X₁₀ is Val or Tyr; X₁₂ is Ser or Ile; X₁₃ is Tyr or Ala; X₁₄ is Leu or Nle; X₁₅ is Asp or Glu; X₁₆ is Arg, Glu, Gly, Lys, or Aib; X₁₇ is Glu, Ile, or Gin; X₁₈ is Ala, Aib, or His; X₁₉ is Ala, Aib, or Gin; X₂₀ is Gin, Glu, or Lys; X₂₃ is Ile or Val; X₂₄ is Ala, Asn, or Gin; X₂₇ is Val or Leu; X₂₈ is Arg or Ala; X₂₉ is Gly or Gin; X₃₀ is Gly or Lys.

In some embodiments, X₁ is Tyr or His; X₂ is Aib or D-Ala; X₁₀ is Val, Tyr, or Y1; X₁₂ is Ser, Ile, or Y1; X₁₃ is Tyr, Ala, or Y1; X₁₄ is Leu, Nle, or Y1; X₁₅ is Asp or Glu; X₁₆ is Arg, Glu, Gly, Lys, Aib, or Y1; X₁₇ is Glu, Ile, Gln, or Y1; X₁₈ is Ala, Aib, or His; X₁₉ is Ala, Aib, or Gin; X₂₀ is Gin, Glu, or Lys; X₂₃ is Ile or Val; X₂₄ is Ala, Asn, or Gin; X₂₇ is Val or Leu; X₂₈ is Arg or Ala; X₂₉ is Gly or Gin; X₃₀ is Gly or Lys; Y1 is a substituted Lys, Orn, Dap, Dab, or Cys residue, for example, a modified group is present on a side chain of the Lys, Orn, Dap, Dab, or Cys residue.

In some embodiments, Y1 is a Lys, Orn, Dap, Dab, or Cys residue with a substituent on a side chain, the substituent having a structure of formula {[2-(2-amino-ethoxy)-ethoxy]-acetyl}ₐ-(y-Glu)_{b}-CO-(CH₂)_{c}-COOH; wherein: a is an integer of 1-3 (may be 1, 2, or 3); b is 1 or 2; c is an integer of 10-30 (may be 10, 11, 12, 13, 14, 15, 16, 17, 18, 19, 20, 21, 22, 23, 24, 25, 26, 27, 28, 29, or 30).

In some embodiments, X₁ is Tyr; X₂ is Aib; X₁₀ is Tyr; X₁₂ is Ile; X₁₃ is Tyr; X₁₄ is Y1; X₁₅ is Asp or Glu; X₁₆ is Arg or Lys; X₁₇ is Ile; X₁₈ is Ala; X₁₉ is Ala; X₂₀ is Gin; X₂₃ is Ile or Val; X₂₄ is Asn; X₂₇ is Ile or Leu; X₂₈ is Ala; X₂₉ is Gly; X₃₀ is Gly; Y1 is a Lys, Orn, Dap, Dab, or Cys residue with a side chain connected to a substituent of formula {[2-(2-amino-ethoxy)-ethoxy]-acetyl}ₐ-(y-Glu)_{b}-CO-(CH₂)_{c}-COOH; a is an integer of 1-3; b is 1 or 2; c is an integer of 10-30.

In some embodiments, X₁ is Tyr; X₂ is Aib; X₁₀ is Tyr; X₁₂ is Ile; X₁₃ is Tyr; X₁₄ is Y1; X₁₅ is Asp or Glu; X₁₆ is Lys; X₁₇ is Ile; X₁₈ is Ala; X₁₉ is Ala; X₂₀ is Gin; X₂₃ is Val; X₂₄ is Asn; X₂₇ is Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(y-Glu)_{b}-CO-(CH₂)_{c}-COOH; a is an integer of 1-3; b is 1 or 2; c is an integer of 10-30.

In some embodiments, X₁ is Tyr; X₂ is Aib or D-Ala; X₁₀ is Y1; X₁₂ is Ile; X₁₃ is Tyr; X₁₄ is Leu or Nle; X₁₅ is Glu; X₁₆ is Arg or Lys; X₁₇ is Ile; X₁₈ is Ala; X₁₉ is Ala; X₂₀ is Gln or Lys; X₂₃ is Ile or Val; X₂₄ is Asn or Gin; X₂₇ is Ile or Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(y-Glu)_{b}-CO-(CH₂)_{c}-COOH, wherein a is an integer of 1-3, b is 1 or 2, and c is an integer of 10-30.

In some embodiments, X₁ is Tyr; X₂ is Aib or D-Ala; X₁₀ is Tyr; X₁₂ is Y1; X₁₃ is Tyr; X₁₄ is Leu or Nle; X₁₅ is Glu; X₁₆ is Arg or Lys; X₁₇ is Ile; X₁₈ is Ala; X₁₉ is Ala; X₂₀ is Gln or Lys; X₂₃ is Ile or Val; X₂₄ is Asn or Gin; X₂₇ is Ile or Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(y-Glu)_{b}-CO-(CH₂)_{c}-COOH, wherein a is an integer of 1-3, b is 1 or 2, and c is an integer of 10-30.

In some embodiments, X₁ is Tyr; X₂ is Aib or D-Ala; X₁₀ is Tyr; X₁₂ is Ile; X₁₃ is Y1; X₁₄ is Leu or Nle; X₁₅ is Glu; X₁₆ is Arg or Lys; X₁₇ is Ile; X₁₈ is Ala; X₁₉ is Ala; X₂₀ is Gln or Lys; X₂₃ is Ile or Val; X₂₄ is Asn or Gin; X₂₇ is Ile or Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(y-Glu)_{b}-CO-(CH₂)_{c}-COOH, wherein a is an integer of 1-3, b is 1 or 2, and c is an integer of 10-30.

In some embodiments, X₁ is Tyr; X₂ is Aib or D-Ala; X₁₀ is Tyr; X₁₂ is Ile; X₁₃ is Tyr; X₁₄ is Y1; X₁₅ is Glu; X₁₆ is Arg or Lys; X₁₇ is Ile; X₁₈ is Ala; X₁₉ is Ala; X₂₀ is Gln or Lys; X₂₃ is Ile or Val; X₂₄ is Asn or Gin; X₂₇ is Ile or Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(y-Glu)_{b}-CO-(CH₂)_{c}-COOH, wherein a is an integer of 1-3, b is 1 or 2, and c is an integer of 10-30.

In some embodiments, X₁ is Tyr; X₂ is Aib; X₁₀ is Tyr; X₁₂ is Ile; X₁₃ is Tyr; X₁₄ is Y1; X₁₅ is Glu; X₁₆ is Arg or Lys; X₁₇ is Ile; X₁₈ is Ala; X₁₉ is Ala; X₂₀ is Gin; X₂₃ is Ile or Val; X₂₄ is Asn; X₂₇ is Ile or Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(y-Glu)_{b}-CO-(CH₂)_{c}-COOH; a is an integer of 1-3; b is 1 or 2; c is an integer of 10-30.

In some embodiments, X₁ is Tyr; X₂ is Aib or D-Ala; X₁₀ is Tyr; X₁₂ is Ile; X₁₃ is Tyr; X₁₄ is Leu or Nle; X₁₅ is Glu; X₁₆ is Y1; X₁₇ is Ile; X₁₈ is Ala; X₁₉ is Ala; X₂₀ is Gln or Lys; X₂₃ is Ile or Val; X₂₄ is Asn or Gin; X₂₇ is Ile or Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(y-Glu)_{b}-CO-(CH₂)_{c}-COOH, wherein a is an integer of 1-3, b is 1 or 2, and c is an integer of 10-30.

In some embodiments, X₁ is Tyr; X₂ is Aib or D-Ala; X₁₀ is Tyr; X₁₂ is Ile; X₁₃ is Tyr; X₁₄ is Leu or Nle; X₁₅ is Glu; X₁₆ is Arg or Lys; X₁₇ is Y1; X₁₈ is Ala; X₁₉ is Ala; X₂₀ is Gln or Lys; X₂₃ is Ile or Val; X₂₄ is Asn or Gin; X₂₇ is Ile or Leu; X₂₈ is Ala; X₂₉ is Gly; X₃₀ is Gly; Y1 is a Lys, Orn, Dap, Dab, or Cys residue with a substituent on a side chain, the substituent having a structure of formula {[2-(2-amino-ethoxy)-ethoxy]-acetyl}ₐ-(y-Glu)_{b}-CO-(CH₂)_{c}-COOH, wherein a is an integer of 1-3, b is 1 or 2, and c is an integer of 10-30.

In some embodiments, X₁₀, X₁₂, X₁₃, X₁₄, X₁₆, and X₁₇ are each independently selected from Y1, wherein Y1 is a Lys, Orn, Dap, Dab, or Cys residue with a substituent on a side chain, the substituent having a structure of formula {[2-(2-amino-ethoxy)-ethoxy]-acetyl}ₐ-(y-Glu)_{b}-CO-(CH₂)_{c}-COOH; a is an integer of 1-3; b is 1 or 2; c is an integer of 10-30.

In some embodiments, a is 2, b is 1 or 2, and c is an integer of 16-20 (e.g., c is 16, 17, 18, 19, or 20).

In some embodiments, a is 2, b is 1 or 2, and c is 16, 18, or 20.

In some embodiments, X₁₀ is Y1; Y1 is Lys with a substituent on a side chain, the substituent having a structure of formula {[2-(2-amino-ethoxy)-ethoxy]-acetyl}ₐ-(y-Glu)_{b}-CO-(CH₂)_{c}-COOH; a is 2; b is 1 or 2; c is 16 or 18.

In some embodiments, X₁₂ is Y1; Y1 is Lys with a substituent on a side chain, the substituent having a structure of formula {[2-(2-amino-ethoxy)-ethoxy]-acetyl}ₐ-(y-Glu)_{b}-CO-(CH₂)_{c}-COOH; a is 2; b is 1 or 2; c is 16 or 18.

In some embodiments, X₁₃ is Y1; Y1 is Lys with a substituent on a side chain, the substituent having a structure of formula {[2-(2-amino-ethoxy)-ethoxy]-acetyl}ₐ-(y-Glu)_{b}-CO-(CH₂)_{c}-COOH; a is 2; b is 1 or 2; c is 16 or 18.

In some embodiments, X₁₄ is Y1; Y1 is Lys with a substituent on a side chain, the substituent having a structure of formula {[2-(2-amino-ethoxy)-ethoxy]-acetyl}ₐ-(y-Glu)_{b}-CO-(CH₂)_{c}-COOH; a is 2; b is 1 or 2; c is 16 or 18.

In some embodiments, X₁₆ is Y1; Y1 is Lys with a substituent on a side chain, the substituent having a structure of formula {[2-(2-amino-ethoxy)-ethoxy]-acetyl}ₐ-(y-Glu)_{b}-CO-(CH₂)_{c}-COOH; a is 2; b is 1 or 2; c is 16 or 18.

In some embodiments, X₁₇ is Y1; Y1 is Lys with a substituent on a side chain, the substituent having a structure of formula {[2-(2-amino-ethoxy)-ethoxy]-acetyl}ₐ-(y-Glu)_{b}-CO-(CH₂)_{c}-COOH; a is 2; b is 1 or 2; c is 16 or 18.

In some embodiments, the side chain amino of the Lys residue in Y1 is covalently connected to a substituent by formation of an amide bond.

In some embodiments, Y1 is K(-OEG-OEG-yGlu-C18-OH) or K(-OEG-OEG-yGlu-C20-OH), wherein K(-OEG-OEG-yGlu-C18-OH) has a structure shown below: 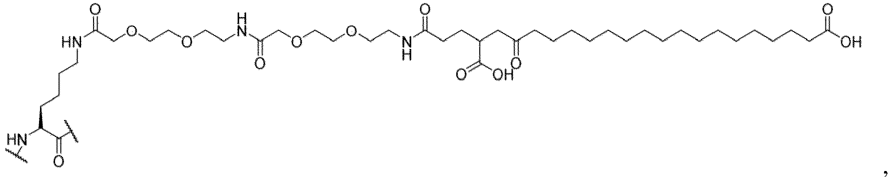 and K(-OEG-OEG-yGlu-C20-OH) has a structure shown below:

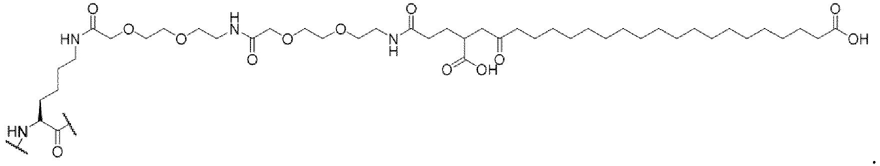

In some embodiments, Y1 is K(-OEG-OEG-yGlu-C18-OH) or K(-OEG-OEG-yGlu-C20-OH), wherein:
K(-OEG-OEG-yGlu-C18-OH) has a structure shown below: 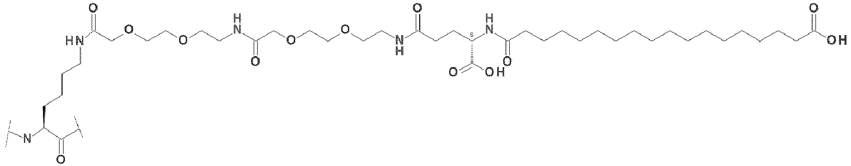 and,
K(-OEG-OEG-yGlu-C20-OH) has a structure shown below:

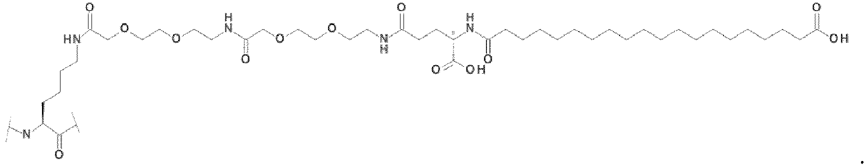

In some embodiments, in Y1, the ε amino of the Lys residue is covalently connected to a substituent by an amide bond, and the α amino of the Lys residue is connected to a peptide chain.

In some embodiments, X₁ is selected from Tyr; X₂ is selected from Aib; X₁₀ is selected from Tyr; X₁₂ is selected from Ile; X₁₃ is selected from Tyr; X₁₄ is selected from Y1; X₁₅ is selected from the group consisting of Asp and Glu; X₁₆ is selected from the group consisting of Arg and Lys; X₁₇ is selected from Ile; X₁₈ is selected from Ala; X₁₉ is selected from Ala; X₂₀ is selected from Gin; X₂₃ is selected from Ile or Val; X₂₄ is selected from Asn; X₂₇ is selected from the group consisting of Ile and Leu; X₂₈ is selected from Ala; X₂₉ is selected from Gly; X₃₀ is selected from Gly; Y1 is K(-OEG-OEG-yGlu-C18-OH) or K(-OEG-OEG-yGlu-C20-OH), wherein
K(-OEG-OEG-yGlu-C18-OH) has a structure shown below: 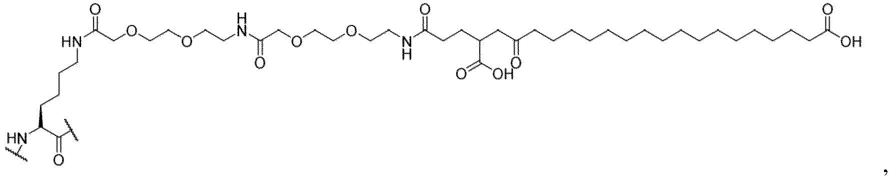
and K(-OEG-OEG-yGlu-C20-OH) has a structure shown below:

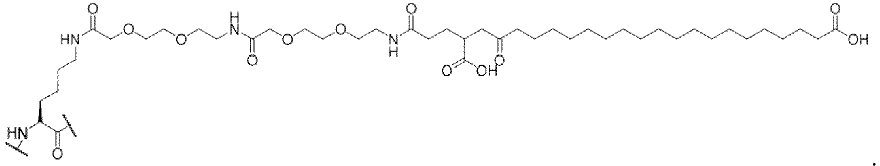

In some embodiments, X₁ is selected from Tyr; X₂ is selected from Aib; X₁₀ is selected from Tyr; X₁₂ is selected from Ile; X₁₃ is selected from Tyr; X₁₄ is selected from Y1; X₁₅ is selected from the group consisting of Asp and Glu; X₁₆ is selected from the group consisting of Arg and Lys; X₁₇ is selected from Ile; X₁₈ is selected from Ala; X₁₉ is selected from Ala; X₂₀ is selected from Gin; X₂₃ is selected from Ile or Val; X₂₄ is selected from Asn; X₂₇ is selected from the group consisting of Ile and Leu; X₂₈ is selected from Ala; X₂₉ is selected from Gly; X₃₀ is selected from Gly; Y1 is K(-OEG-OEG-yGlu-C18-OH) or K(-OEG-OEG-yGlu-C20-OH), wherein
K(-OEG-OEG-yGlu-C18-OH) has a structure shown below: 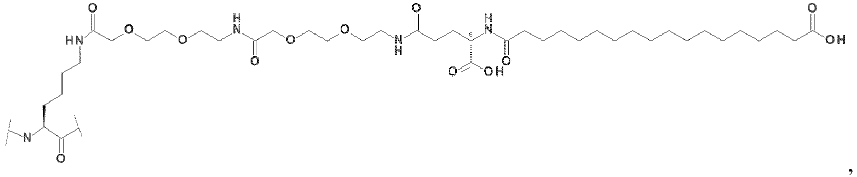 and
K(-OEG-OEG-yGlu-C20-OH) has a structure shown below:

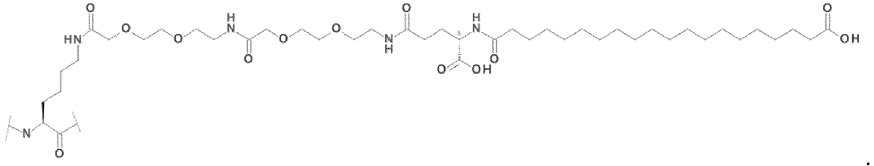

In some embodiments of the present disclosure, the GLP-1 analog is represented by the general formula (II) (SEQ ID NO: 20):
H-YAibEGTFTSDYSIYX₁₄X₁₅X₁₆IAAQEFX₂₃NWLX₂₇AGGPSSGAPPPS-NH₂ (II), wherein X₁₄ is K or L, X₁₅ is D or E, X₁₆ is K or R, X₂₃ is V or I, and X₂₇ is I or L.

In some embodiments of the present disclosure, the GLP-1 analog is selected from the group consisting of the following compounds numbered 1 to 18:

| SEQ ID NO | Sequence |
|---|---|
| 1 | H-YAibEGTFTSDYSIYKDKIAAQEFVNWLIAGGPSSGAPPPS-NH₂ |
| 2 | H-YAibEGTFTSDYSIYKDRIAAQEFVNWLIAGGPSSGAPPPS-NH₂ |
| 3 | H-YAibEGTFTSDYSIYKDKIAAQEFINWLIAGGPSSGAPPPS-NH₂ |
| 4 | H-YAibEGTFTSDYSIYKDRIAAQEFINWLIAGGPSSGAPPPS-NH₂ |
| 5 | H-YAibEGTFTSDYSIYKDKIAAQEFINWLLAGGPSSGAPPPS-NH₂ |
| 6 | H-YAibEGTFTSDYSIYKDRIAAQEFVNWLLAGGPSSGAPPPS-NH₂ |
| 7 | H-YAibEGTFTSDYSIYKDKIAAQEFVNWLLAGGPSSGAPPPS-NH₂ |
| 8 | H-YAibEGTFTSDYSIYLEKIAAQEFVNWLLAGGPSSGAPPPS-NH₂ |
| 9 | H-YAibEGTFTSDYSIYLEKIAAQEFVNWLIAGGPSSGAPPPS-NH₂ |
| 10 | H-YAibEGTFTSDYSIYLEKIAAQEFINWLIAGGPSSGAPPPS-NH₂ |
| 11 | H-YAibEGTFTSDYSIYLEKIAAQEFINWLLAGGPSSGAPPPS-NH₂ |
| 12 | H-YAibEGTFTSDYSIYKEKIAAQEFVNWLIAGGPSSGAPPPS-NH₂ |
| 13 | H-YAibEGTFTSDYSIYKERIAAQEFVNWLIAGGPSSGAPPPS-NH₂ |
| 14 | H-YAibEGTFTSDYSIYKEKIAAQEFINWLIAGGPSSGAPPPS-NH₂ |
| 15 | H-YAibEGTFTSDYSIYKERIAAQEFINWLIAGGPSSGAPPPS-NH₂ |
| 16 | H-YAibEGTFTSDYSIYKEKIAAQEFINWLLAGGPSSGAPPPS-NH₂ |
| 17 | H-YAibEGTFTSDYSIYKERIAAQEFVNWLLAGGPSSGAPPPS-NH₂ |
| 18 | H-YAibEGTFTSDYSIYKEKIAAQEFVNWLLAGGPSSGAPPPS-NH₂. |

In some embodiments of the present disclosure, the GLP-1 analog is selected from the group consisting of the following compounds numbered 1# to 18#:

| No. | Sequence |
|---|---|
| 1# | H-YAibEGTFTSDYSIYK(OEG-OEG-yGlu-C20-OH)DKIAAQEFVNWLIAGGPSSGAPP PS-NH2 |
| 2# | H-YAibEGTFTSDYSIYK(OEG-OEG-yGlu-C20-OH)DRIAAQEFVNWLIAGGPSSGAPP PS-NH2 |
| 3# | H-YAibEGTFTSDYSIYK(OEG-OEG-yGlu-C20-OH)DKIAAQEFINWLIAGGPSSGAPPP S-NH2 |
| 4# | H-YAibEGTFTSDYSIYK(OEG-OEG-yGlu-C20-OH)DRIAAQEFINWLIAGGPSSGAPPP S-NH2 |
| 5# | H-YAibEGTFTSDYSIYK(OEG-OEG-yGlu-C20-OH)DKIAAQEFINWLLAGGPSSGAPP PS-NH2 |
| 6# | H-YAibEGTFTSDYSIYK(OEG-OEG-yGlu-C20-OH)DRIAAQEFVNWLLAGGPSSGAP |
| | PPS-NH2 |
| 7# | H-YAibEGTFTSDYSIYK(OEG-OEG-yGlu-C20-OH)DKIAAQEFVNWLLAGGPSSGAP PPS-NH2 |
| 8# | H-YAibEGTFTSDYSIYLEK(OEG-OEG-γGlu-C20-OH)IAAQEFVNWLLAGGPSSGAPP PS-NH2 |
| 9# | H-YAibEGTFTSDYSIYLEK(OEG-OEG-yGlu-C20-OH)IAAQEFVNWLIAGGPSSGAPP PS-NH2 |
| 10# | H-YAibEGTFTSDYSIYLEK(OEG-OEG-γGlu-C20-OH)IAAQEFINWLIAGGPSSGAPPP S-NH2 |
| 11# | H-YAibEGTFTSDYSIYLEK(OEG-OEG-yGlu-C20-OH)IAAQEFINWLLAGGPSSGAPP PS-NH2 |
| 12# | H-YAibEGTFTSDYSIYK(OEG-OEG-yGlu-C20-OH)EKIAAQEFVNWLIAGGPSSGAPP PS-NH2 |
| 13# | H-YAibEGTFTSDYSIYK(OEG-OEG-yGlu-C20-OH)ERIAAQEFVNWLIAGGPSSGAPP PS-NH2 |
| 14# | H-YAibEGTFTSDYSIYK(OEG-OEG-yGlu-C20-OH)EKIAAQEFINWLIAGGPSSGAPPP S-NH2 |
| 15# | H-YAibEGTFTSDYSIYK(OEG-OEG-yGlu-C20-OH)ERIAAQEFINWLIAGGPSSGAPPP S-NH2 |
| 16# | H-YAibEGTFTSDYSIYK(OEG-OEG-yGlu-C20-OH)EKIAAQEFINWLLAGGPSSGAPP PS-NH2 |
| 17# | H-YAibEGTFTSDYSIYK(OEG-OEG-yGlu-C20-OH)ERIAAQEFVNWLLAGGPSSGAPP PS-NH2 |
| 18# | H-YAibEGTFTSDYSIYK(OEG-OEG-γGlu-C20-OH)EKIAAQEFVNWLLAGGPSSGAPP PS-NH2. |

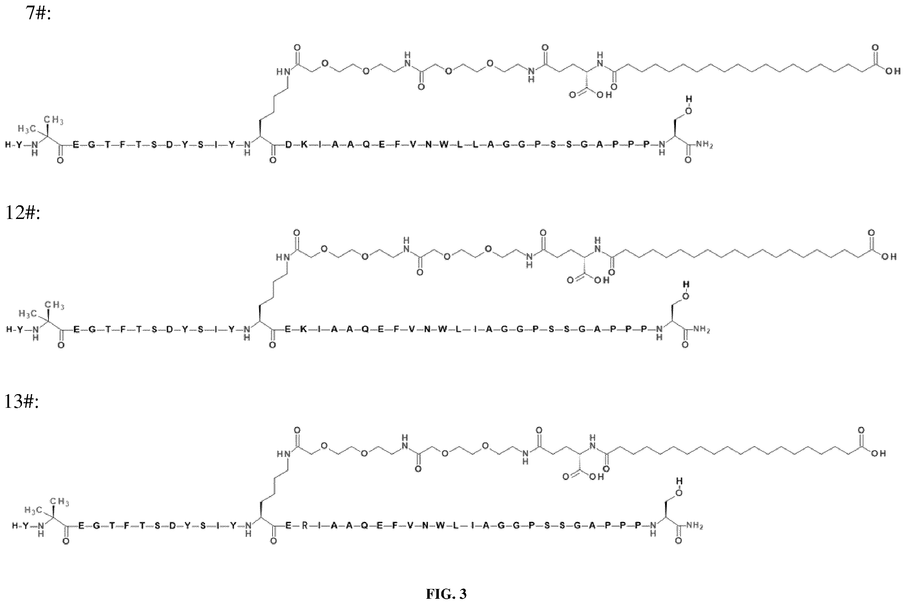

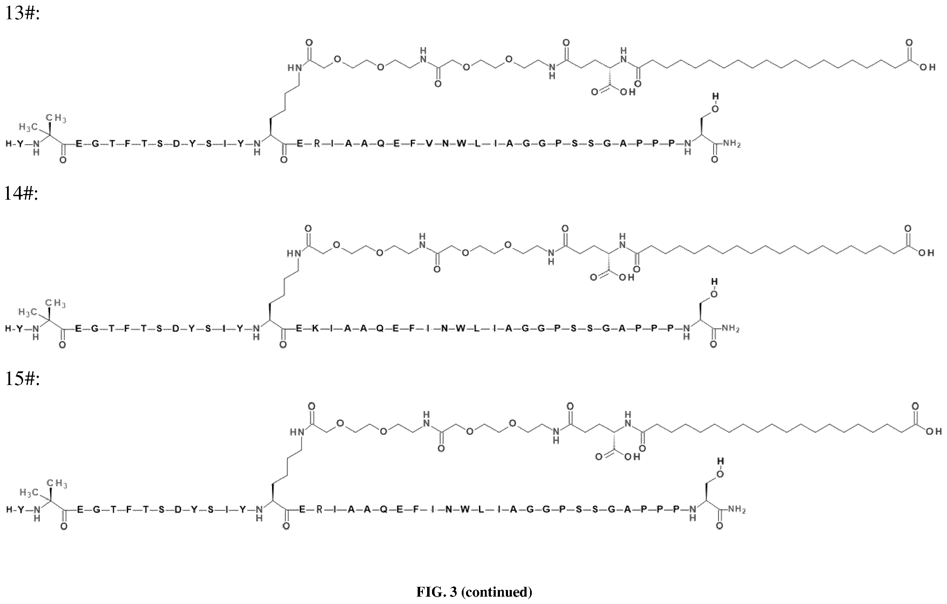

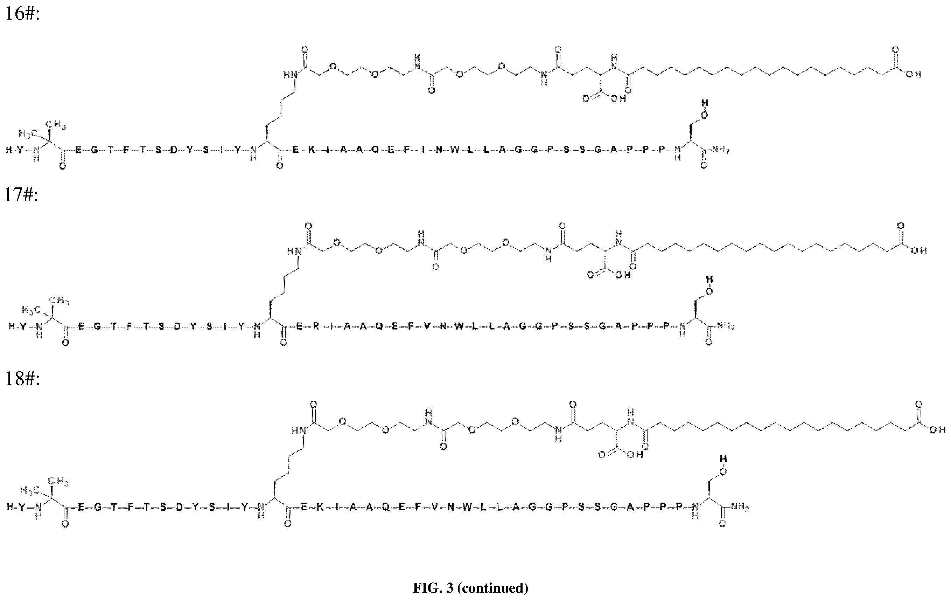

In some embodiments, the GLP-1 analog of the present disclosure is selected from the group consisting of compounds shown as 7#, 12#, 13#, 14#, 15#, 16#, 17#, and 18 # in FIG. 3.

In some embodiments, the concentration of the GLP-1 analog or the pharmaceutically acceptable salt thereof in the pharmaceutical composition is 0.01 mg/mL to 1000 mg/mL, e.g., 0.1 mg/mL to 500 mg/mL, 0.1 mg/mL to 400 mg/mL, 0.1 mg/mL to 300 mg/mL, 0.1 mg/mL to 200 mg/mL, 0.1 mg/mL to 100 mg/mL, 0.5 mg/mL to 200 mg/mL, 0.5 mg/mL to 150 mg/mL, 0.5 mg/mL to 100 mg/mL, 0.5 mg/mL to 50 mg/mL, 0.5 mg/mL to 25 mg/mL, 1.0 mg/mL to 100 mg/mL, 1.0 mg/mL to 90 mg/mL, 1.0 mg/mL to 80 mg/mL, 1.0 mg/mL to 70 mg/mL, 1.0 mg/mL to 60 mg/mL, 1.0 mg/mL to 50 mg/mL, 1.0 mg/mL to 40 mg/mL, 1.0 mg/mL to 30 mg/mL, 1.0 mg/mL to 20 mg/mL, 1.0 mg/mL to 10 mg/mL, 1.0 mg/mL to 9.0 mg/mL, 1.0 mg/mL to 8.0 mg/mL, 1.0 mg/mL to 7.0 mg/mL, 1.0 mg/mL to 6.0 mg/mL, 1.0 mg/mL to 5.0 mg/mL, 2.0 mg/mL to 50 mg/mL, 2.0 mg/mL to 40 mg/mL, 2.0 mg/mL to 30 mg/mL, 2.0 mg/mL to 20 mg/mL, 2.0 mg/mL to 10 mg/mL, 2.0 mg/mL to 9.0 mg/mL, 2.0 mg/mL to 8.0 mg/mL, 2.0 mg/mL to 7.0 mg/mL, 2.0 mg/mL to 6.0 mg/mL, 2.0 mg/mL to 5.0 mg/mL, 5.0 mg/mL to 1000 mg/mL, 5.0 mg/mL to 500 mg/mL, 5.0 mg/mL to 400 mg/mL, 5.0 mg/mL to 300 mg/mL, 5.0 mg/mL to 200 mg/mL, 5.0 mg/mL to 100 mg/mL, 5.0 mg/mL to 90 mg/mL, 5.0 mg/mL to 80 mg/mL, 5.0 mg/mL to 70 mg/mL, 5.0 mg/mL to 60 mg/mL, 5.0 mg/mL to 50 mg/mL, 5.0 mg/mL to 40 mg/mL, 5.0 mg/mL to 30 mg/mL, 5.0 mg/mL to 20 mg/mL, 5.0 mg/mL to 10 mg/mL, 6.0 mg/mL to 1000 mg/mL, 6.0 mg/mL to 500 mg/mL, 6.0 mg/mL to 400 mg/mL, 6.0 mg/mL to 300 mg/mL, 6.0 mg/mL to 200 mg/mL, 6.0 mg/mL to 100 mg/mL, 6.0 mg/mL to 90 mg/mL, 6.0 mg/mL to 80 mg/mL, 6.0 mg/mL to 70 mg/mL, 6.0 mg/mL to 60 mg/mL, 6.0 mg/mL to 50 mg/mL, 6.0 mg/mL to 40 mg/mL, 6.0 mg/mL to 30 mg/mL, 6.0 mg/mL to 20 mg/mL, or 6.0 mg/mL to 10 mg/mL. In some embodiments, the concentration of the GLP-1 analog or the pharmaceutically acceptable salt thereof in the pharmaceutical composition is about 1.0 mg/mL, about 2.0 mg/mL, about 3.0 mg/mL, about 4.0 mg/mL, about 5.0 mg/mL, about 6.0 mg/mL, about 7.0 mg/mL, about 8.0 mg/mL, about 9.0 mg/mL, about 10.0 mg/mL, about 11.0 mg/mL, about 12.0 mg/mL, about 13.0 mg/mL, about 14.0 mg/mL, about 15.0 mg/mL, about 16.0 mg/mL, about 17.0 mg/mL, about 18.0 mg/mL, about 19.0 mg/mL, about 20.0 mg/mL, about 21.0 mg/mL, about 22.0 mg/mL, about 23.0 mg/mL, about 24.0 mg/mL, about 25.0 mg/mL, about 26.0 mg/mL, about 27.0 mg/mL, about 28.0 mg/mL, about 29.0 mg/mL, or about 30.0 mg/mL. In some embodiments, the concentration of the GLP-1 analog or the pharmaceutically acceptable salt thereof in the pharmaceutical composition is about 2.0 mg/mL or about 10.0 mg/mL.

In some embodiments, the pharmaceutical composition further comprises an osmotic pressure regulator. The osmotic pressure regulator includes, but is not limited to: salts (e.g., sodium chloride, phosphate, sodium citrate, boric acid, and sodium tartrate), sugar or sugar alcohol (lactose, trehalose, sucrose, glucose, mannitol, sorbitol, and xylitol), amino acids (e.g., L-glycine, L-histidine, arginine, lysine, isoleucine, aspartic acid, tryptophan, and threonine), polyhydric sugar alcohol (e.g., glycerol, 1,2-propanediol (also referred to herein as propylene glycol), 1,3-propanediol, and 1,3-butanediol), polyethylene glycol (e.g., PEG 400), or mixtures thereof.

In some embodiments, the osmotic pressure regulator is selected from one or more of propylene glycol, mannitol, sorbitol, xylitol, glycerol, lactose, trehalose, sucrose, glucose, sodium chloride, phosphate, sodium citrate, boric acid, and sodium tartrate.

In some embodiments, the osmotic pressure regulator is sodium chloride.

In some embodiments, the osmotic pressure regulator is propylene glycol or mannitol.

In some embodiments, the concentration of propylene glycol in the pharmaceutical composition is 10 mg/mL to 20 mg/mL, e.g., 10 mg/mL to 19 mg/mL, 10 mg/mL to 18 mg/mL, 10 mg/mL to 17 mg/mL, 10 mg/mL to 16 mg/mL, 10 mg/mL to 15 mg/mL, 11 mg/mL to 20 mg/mL, 11 mg/mL to 19 mg/mL, 11 mg/mL to 18 mg/mL, 11 mg/mL to 17 mg/mL, 11 mg/mL to 16 mg/mL, 11 mg/mL to 15 mg/mL, 12 mg/mL to 20 mg/mL, 12 mg/mL to 19 mg/mL, 12 mg/mL to 18 mg/mL, 12 mg/mL to 17 mg/mL, 12 mg/mL to 16 mg/mL, 12 mg/mL to 15 mg/mL, 13 mg/mL to 20 mg/mL, 13 mg/mL to 19 mg/mL, 13 mg/mL to 18 mg/mL, 13 mg/mL to 17 mg/mL, 13 mg/mL to 16 mg/mL, or 13 mg/mL to 15 mg/mL. In some embodiments, the concentration of propylene glycol in the pharmaceutical composition is 12 mg/mL to 16 mg/mL, e.g., about 14 mg/mL.

In some embodiments, the concentration of sodium chloride in the pharmaceutical composition is 1 mg/mL to 20 mg/mL, e.g., 1 mg/mL to 19 mg/mL, 1 mg/mL to 18 mg/mL, 1 mg/mL to 17 mg/mL, 1 mg/mL to 16 mg/mL, 1 mg/mL to 15 mg/mL, 2 mg/mL to 18 mg/mL, 2 mg/mL to 17 mg/mL, 2 mg/mL to 16 mg/mL, 2 mg/mL to 15 mg/mL, 3 mg/mL to 18 mg/mL, 3 mg/mL to 17 mg/mL, 3 mg/mL to 16 mg/mL, 3 mg/mL to 15 mg/mL, 4 mg/mL to 14 mg/mL, 5 mg/mL to 13 mg/mL, 6 mg/mL to 12 mg/mL, 7 mg/mL to 11 mg/mL, 7 mg/mL to 10.5 mg/mL, 7 mg/mL to 10 mg/mL, 7 mg/mL to 9 mg/mL, 7 mg/mL to 9.5 mg/mL, 7 mg/mL to 9 mg/mL, 7 mg/mL to 8.5 mg/mL, 7.5 mg/mL to 11 mg/mL, 7.5 mg/mL to 10.5 mg/mL, 7.5 mg/mL to 10 mg/mL, 7.5 mg/mL to 9.5 mg/mL, 7.5 mg/mL to 9.0 mg/mL, 7.5 mg/mL to 8.5 mg/mL, 8 mg/mL to 11 mg/mL, 8 mg/mL to 10.5 mg/mL, 8 mg/mL to 10 mg/mL, 8 mg/mL to 9.5 mg/mL, or 8 mg/mL to 9 mg/mL. In some embodiments, the concentration of sodium chloride in the pharmaceutical composition is 2 mg/mL to 18 mg/mL. In some embodiments, the concentration of sodium chloride in the pharmaceutical composition is 3 mg/mL to 15 mg/mL. In some embodiments, the concentration of sodium chloride in the pharmaceutical composition is 7 mg/mL to 10 mg/mL, e.g., about 7.5 mg/mL, about 7.6 mg/mL, about 7.7 mg/mL, about 7.8 mg/mL, about 7.9 mg/mL, about 8 mg/mL, about 8.1 mg/mL, about 8.2 mg/mL, about 8.3 mg/mL, about 8.4 mg/mL, about 8.5 mg/mL, about 8.6 mg/mL, about 8.7 mg/mL, about 8.8 mg/mL, about 8.9 mg/mL, about 9.0 mg/mL, about 9.1 mg/mL, about 9.28 mg/mL, about 9.3 mg/mL, about 9.4 mg/mL, about 9.5 mg/mL, about 9.6 mg/mL, about 9.7 mg/mL, about 9.8 mg/mL, about 9.9 mg/mL, or about 10 mg/mL. In some embodiments, the concentration of sodium chloride in the pharmaceutical composition is 7.5 mg/mL to 9.5 mg/mL.

In some embodiments, the concentration of mannitol in the pharmaceutical composition is 10 mg/mL to 50 mg/mL, e.g., 15 mg/mL to 45 mg/mL, 20 mg/mL to 45 mg/mL, 25 mg/mL to 45 mg/mL, 30 mg/mL to 45 mg/mL, 15 mg/mL to 40 mg/mL, 15 mg/mL to 35 mg/mL, 20 mg/mL to 40 mg/mL, 20 mg/mL to 35 mg/mL, 25 mg/mL to 40 mg/mL, 25 mg/mL to 35 mg/mL, 30 mg/mL to 40 mg/mL, or 35 mg/mL to 40 mg/mL. In some embodiments, the concentration of mannitol in the pharmaceutical composition is about 25 mg/mL, 25.5 mg/mL, about 26 mg/mL, 26.5 mg/mL, about 27 mg/mL, 27.5 mg/mL, about 28 mg/mL, 28.5 mg/mL, about 29 mg/mL, 29.5 mg/mL, about 30 mg/mL, 30.5 mg/mL, about 31 mg/mL, 31.5 mg/mL, about 32 mg/mL, 32.5 mg/mL, about 33 mg/mL, 33.5 mg/mL, about 34 mg/mL, 34.5 mg/mL, about 35 mg/mL, 35.5 mg/mL, about 36 mg/mL, 36.5 mg/mL, about 37 mg/mL, 37.5 mg/mL, about 38 mg/mL, 38.5 mg/mL, about 39 mg/mL, 39.5 mg/mL, or about 40 mg/mL.

In some embodiments, the concentration of the buffer in the pharmaceutical composition is 0.5 mM to 50.0 mM, e.g., 0.5 mM to 40.0 mM, 0.5 mM to 30.0 mM, 0.5 mM to 20.0 mM, 0.5 mM to 10.0 mM, 1.0 mM to 40.0 mM, 1.0 mM to 35.0 mM, 1.0 mM to 30.0 mM, 1.0 mM to 25.0 mM, 1.0 mM to 20.0 mM, 1.0 mM to 15.0 mM, 1.0 mM to 10.0 mM, 2.0 mM to 40.0 mM, 2.0 mM to 35.0 mM, 2.0 mM to 30.0 mM, 2.0 mM to 25.0 mM, 2.0 mM to 20.0 mM, 2.0 mM to 15.0 mM, 2.0 mM to 10.0 mM, or 5.0 mM to 10.0 mM. In some embodiments, the concentration of the buffer in the pharmaceutical composition is about 1.0 mM, about 2.0 mM, about 3.0 mM, about 4.0 mM, about 5.0 mM, about 6.0 mM, about 7.0 mM, about 8.0 mM, about 9.0 mM, or about 10.0 mM. In some embodiments, the concentration of the buffer in the pharmaceutical composition is about 5.0 mM.

In some embodiments, the pharmaceutical composition further comprises a pH adjuster, such as sodium hydroxide and/or hydrochloric acid.

In some embodiments, the pH of the pharmaceutical composition is 6.5 to 9.0, e.g., 6.6 to 9.0, 6.7 to 9.0, 6.8 to 9.0, 6.9 to 9.0, 7.0 to 9.0, 7.1 to 9.0, 7.2 to 9.0, 7.3 to 9.0, 7.4 to 9.0, 7.5 to 9.0, 7.6 to 9.0, 7.7 to 9.0, 7.8 to 9.0, 7.9 to 9.0, 8.0 to 9.0, 7.0 to 8.9, 7.0 to 8.8, 7.0 to 8.7, 7.0 to 8.6, 7.0 to 8.5, 7.0 to 8.4, 7.0 to 8.3, 7.0 to 8.2, 7.0 to 8.1, 7.0 to 8.0, 7.1 to 8.9, 7.1 to 8.8, 7.1 to 8.7, 7.1 to 8.6, 7.1 to 8.5, 7.1 to 8.4, 7.1 to 8.3, 7.1 to 8.2, 7.1 to 8.1, 7.1 to 8.0, 7.1 to 7.9, 7.1 to 7.8, or 7.1 to 7.7. In some embodiments, the pH of the pharmaceutical composition is about 7.0, about 7.1, about 7.2, about 7.3, about 7.4, about 7.5, about 7.6, about 7.7, about 7.8, about 7.9, about 8.0, about 8.1, about 8.2, about 8.3, about 8.4, or about 8.5. In some embodiments, the pH of the pharmaceutical composition is 7.0 to 8.0. In some embodiments, the pH of the pharmaceutical composition is 7.1 to 7.7, e.g., about 7.5 or about 7.4.

In some embodiments, the pharmaceutical composition further comprises a pharmaceutically acceptable bacteriostatic agent.

In some embodiments, the pharmaceutically acceptable bacteriostatic agent includes, but is not limited to, phenol, *o*-cresol, *m*-cresol, *p*-cresol, methyl paraben, propyl paraben, 2-phenoxyethanol, butyl paraben, 2-phenylethyl alcohol, benzyl alcohol, ethanol, chlorobutanol, thimerosal, bronopol, benzoic acid, imidurea, chlorhexidine, sodium dehydroacetate, chlorocresol, ethyl paraben, benzethonium chloride, or mixtures thereof.

In some embodiments, the pharmaceutically acceptable bacteriostatic agent is phenol. In some embodiments, the concentration of the bacteriostatic agent in the pharmaceutical composition is 4.0 mg/mL-7.0 mg/mL, e.g., 4.2 mg/mL-6.9 mg/mL, 4.4 mg/mL-6.9 mg/mL, 4.6 mg/mL-6.9 mg/mL, 4.8 mg/mL-6.9 mg/mL, 5.0 mg/mL-6.9 mg/mL, 5.1 mg/mL-6.9 mg/mL, 5.2 mg/mL-6.9 mg/mL, 5.3 mg/mL-6.9 mg/mL, 5.4 mg/mL-6.9 mg/mL, 5.5 mg/mL-6.9 mg/mL, 4.2 mg/mL-6.8 mg/mL, 4.4 mg/mL-6.8 mg/mL, 4.6 mg/mL-6.8 mg/mL, 4.8 mg/mL-6.8 mg/mL, 5.0 mg/mL-6.8 mg/mL, 5.1 mg/mL-6.8 mg/mL, 5.2 mg/mL-6.8 mg/mL, 5.3 mg/mL-6.8 mg/mL, 5.4 mg/mL-6.8 mg/mL, 5.5 mg/mL-6.8 mg/mL, 4.4 mg/mL-6.7 mg/mL, 4.6 mg/mL-6.7 mg/mL, 4.8 mg/mL-6.7 mg/mL, 5.0 mg/mL-6.7 mg/mL, 5.1 mg/mL-6.7 mg/mL, 5.2 mg/mL-6.7 mg/mL, 5.3 mg/mL-6.7 mg/mL, 5.4 mg/mL-6.7 mg/mL, 5.5 mg/mL-6.7 mg/mL, 4.4 mg/mL-6.6 mg/mL, 4.6 mg/mL-6.6 mg/mL, 4.8 mg/mL-6.6 mg/mL, 5.0 mg/mL-6.6 mg/mL, 5.1 mg/mL-6.6 mg/mL, 5.2 mg/mL-6.6 mg/mL, 5.3 mg/mL-6.6 mg/mL, 5.4 mg/mL-6.6 mg/mL, or 5.5 mg/mL-6.6 mg/mL. In some embodiments, the concentration of the bacteriostatic agent is 4.2 mg/mL-6.9 mg/mL. In some embodiments, the concentration of the bacteriostatic agent is 5.5 mg/mL-6.6 mg/mL, e.g., about 5.5 mg/mL.

In some embodiments, the pharmaceutical composition comprises any one of the following groups 1) to 6):
1) the compound shown as 18# in FIG. 3 or the pharmaceutically acceptable salt thereof;
   a buffer, such as a phosphate buffer, e.g., sodium dihydrogen phosphate;
   propylene glycol or mannitol; and
   optionally, an antibacterial agent, such as phenol;
2) the compound shown as 18# in FIG. 3 or the pharmaceutically acceptable salt thereof;
   a buffer, such as a phosphate buffer, e.g., sodium dihydrogen phosphate;
   sodium chloride; and
   optionally, an antibacterial agent, such as phenol;
3) the compound shown as 18# in FIG. 3 or the pharmaceutically acceptable salt thereof;
   a buffer, such as a phosphate buffer, e.g., sodium dihydrogen phosphate;
   propylene glycol or mannitol;
   optionally, an antibacterial agent, such as phenol; and
   a pH adjuster, such as sodium hydroxide and/or hydrochloric acid;
4) the compound shown as 18# in FIG. 3 or the pharmaceutically acceptable salt thereof;
   a buffer, such as a phosphate buffer, e.g., sodium dihydrogen phosphate;
   sodium chloride;
   optionally, an antibacterial agent, such as phenol; and
   a pH adjuster, such as sodium hydroxide and/or hydrochloric acid;
5) the compound shown as 18# in FIG. 3 or the pharmaceutically acceptable salt thereof;
   a buffer, such as a phosphate buffer, e.g., sodium dihydrogen phosphate;
   propylene glycol or mannitol;
   optionally, an antibacterial agent, such as phenol;
   a pH adjuster, such as sodium hydroxide and/or hydrochloric acid; and
   water for injection;
6) the compound shown as 18# in FIG. 3 or the pharmaceutically acceptable salt thereof;
   a buffer, such as a phosphate buffer, e.g., sodium dihydrogen phosphate;
   sodium chloride;
   optionally, an antibacterial agent, such as phenol;
   a pH adjuster, such as sodium hydroxide and/or hydrochloric acid; and
   water for injection.

In some embodiments, the pharmaceutical composition consists of: the compound shown as 18# in FIG. 3 or the pharmaceutically acceptable salt thereof; sodium dihydrogen phosphate; propylene glycol or mannitol; phenol; sodium hydroxide and/or hydrochloric acid; and water for injection.

In some embodiments, the pharmaceutical composition consists of: the compound shown as 18# in FIG. 3 or the pharmaceutically acceptable salt thereof; sodium dihydrogen phosphate; propylene glycol or mannitol; sodium hydroxide and/or hydrochloric acid; and water for injection.

In some embodiments, the pharmaceutical composition consists of: the compound shown as 18# in FIG. 3 or the pharmaceutically acceptable salt thereof; sodium dihydrogen phosphate; sodium chloride; phenol; sodium hydroxide and/or hydrochloric acid; and water for injection.

In some embodiments, the pharmaceutical composition consists of: the compound shown as 18# in FIG. 3 or the pharmaceutically acceptable salt thereof; sodium dihydrogen phosphate; sodium chloride; sodium hydroxide and/or hydrochloric acid; and water for injection.

In some embodiments, the pharmaceutical composition comprises any one of A to K:
A) 1.0 mg/mL to 100 mg/mL compound shown as 18# in FIG. 3 or the pharmaceutically acceptable salt thereof,
   1.0 mM to 35.0 mM sodium dihydrogen phosphate, and
   10 mg/mL to 20 mg/mL propylene glycol or 10 mg/mL to 50 mg/mL mannitol;
   the pH of the pharmaceutical composition is 6.5 to 9.0;
B) 1.0 mg/mL to 100 mg/mL compound shown as 18# in FIG. 3 or the pharmaceutically acceptable salt thereof,
   1.0 mM to 35.0 mM sodium dihydrogen phosphate, and
   2 mg/mL to 18 mg/mL sodium chloride;
   the pH of the pharmaceutical composition is 6.5 to 9.0;
C) 1.0 mg/mL to 100 mg/mL compound shown as 18# in FIG. 3 or the pharmaceutically acceptable salt thereof,
   1.0 mM to 35.0 mM sodium dihydrogen phosphate,
   10 mg/mL to 20 mg/mL propylene glycol, or 15 mg/mL to 45 mg/mL mannitol, or 2 mg/mL to 18 mg/mL sodium chloride, and
   optionally, an antibacterial agent, such as 4.0 mg/mL to 7.0 mg/mL phenol;
   the pH of the pharmaceutical composition is 6.5 to 9.0;
D) 1.0 mg/mL to 30.0 mg/mL compound shown as 18# in FIG. 3 or the pharmaceutically acceptable salt thereof,
   1.0 mM to 25.0 mM sodium dihydrogen phosphate,
   11 mg/mL to 18 mg/mL propylene glycol, or 20 mg/mL to 40 mg/mL mannitol, or 3 mg/mL to 15 mg/mL sodium chloride, and
   optionally, an antibacterial agent, such as 4.2 mg/mL to 6.9 mg/mL phenol;
   the pH of the pharmaceutical composition is 7.0 to 8.0;
E) 2.0 mg/mL to 10.0 mg/mL compound shown as 18# in FIG. 3 or the pharmaceutically acceptable salt thereof,
   2.0 mM to 10.0 mM sodium dihydrogen phosphate,
   12 mg/mL to 16 mg/mL propylene glycol, or 25 mg/mL to 35 mg/mL mannitol, or 8 mg/mL to 10 mg/mL sodium chloride, and
   optionally, an antibacterial agent, such as 4.4 mg/mL to 6.8 mg/mL phenol;
   the pH of the pharmaceutical composition is 7.1 to 7.7;
F) 1.0 mg/mL to 100 mg/mL compound shown as 18# in FIG. 3 or the pharmaceutically acceptable salt thereof,
   1.0 mM to 35.0 mM sodium dihydrogen phosphate,
   10 mg/mL to 20 mg/mL propylene glycol, or 15 mg/mL to 45 mg/mL mannitol, or 2 mg/mL to 18 mg/mL sodium chloride,
   optionally, an antibacterial agent, such as 4.0 mg/mL to 7.0 mg/mL phenol, and
   water for injection;
   the pH of the pharmaceutical composition is 6.5 to 9.0;
G) 1.0 mg/mL to 30.0 mg/mL compound shown as 18# in FIG. 3 or the pharmaceutically acceptable salt thereof,
   1.0 mM to 25.0 mM sodium dihydrogen phosphate,
   11 mg/mL to 18 mg/mL propylene glycol, or 20 mg/mL to 40 mg/mL mannitol, or 3 mg/mL to 15 mg/mL sodium chloride,
   optionally, an antibacterial agent, such as 4.2 mg/mL to 6.9 mg/mL phenol, and
   water for injection;
   the pH of the pharmaceutical composition is 7.0 to 8.0;
H) 2.0 mg/mL to 20.0 mg/mL compound shown as 18# in FIG. 3 or the pharmaceutically acceptable salt thereof,
   2.0 mM to 10.0 mM sodium dihydrogen phosphate,
   12 mg/mL to 16 mg/mL propylene glycol, or 25 mg/mL to 35 mg/mL mannitol, or 7 mg/mL to 10 mg/mL sodium chloride,
   optionally, an antibacterial agent, such as 4.4 mg/mL to 6.8 mg/mL phenol, and
   water for injection;
   the pH of the pharmaceutical composition is 7.1 to 7.7;
I) 5.0 mg/mL to 15.0 mg/mL or 5.0 mg/mL to 10.0 mg/mL compound shown as 18# in FIG. 3 or the pharmaceutically acceptable salt thereof,
   5.0 mM to 10.0 mM sodium dihydrogen phosphate,
   12 mg/mL to 16 mg/mL propylene glycol, or 7 mg/mL to 10 mg/mL sodium chloride, or
   8 mg/mL to 9 mg/mL sodium chloride,
   optionally, an antibacterial agent, such as 4.4 mg/mL to 6.8 mg/mL phenol, and
   water for injection;
   the pH of the pharmaceutical composition is 7.1 to 7.7;
J) 5.0 mg/mL to 15.0 mg/mL or 5.0 mg/mL to 10.0 mg/mL compound shown as 18# in FIG. 3 or the pharmaceutically acceptable salt thereof,
   4.0 mM to 8.0 mM or 4.0 mM to 6.0 mM sodium dihydrogen phosphate,
   12 mg/mL to 16 mg/mL propylene glycol, or 7 mg/mL to 10 mg/mL sodium chloride, or
   8 mg/mL to 9 mg/mL sodium chloride,
   optionally, an antibacterial agent, such as 4.4 mg/mL to 6.8 mg/mL phenol, and
   water for injection;
   the pH of the pharmaceutical composition is 7.1 to 7.7;
K) 1.0 mg/mL to 20.0 mg/mL compound shown as 18# in FIG. 3 or the pharmaceutically acceptable salt thereof,
   1.0 mM to 25.0 mM sodium dihydrogen phosphate,
   11 mg/mL to 18 mg/mL propylene glycol, or 20 mg/mL to 40 mg/mL mannitol, or 3 mg/mL to 15 mg/mL sodium chloride, and
   optionally, an antibacterial agent, such as 4.2 mg/mL to 6.9 mg/mL phenol;
   the pH of the pharmaceutical composition is 7.0 to 8.0.

In some embodiments, the pharmaceutical composition comprises:
6.0 mg/mL to 10.0 mg/mL compound shown as 18# in FIG. 3 or the pharmaceutically acceptable salt thereof,
2.0 mM to 10.0 mM sodium dihydrogen phosphate, and
30 mg/mL to 40 mg/mL mannitol.

In some embodiments, the pharmaceutical composition comprises:
6.0 mg/mL to 10.0 mg/mL compound shown as 18# in FIG. 3 or the pharmaceutically acceptable salt thereof,
2.0 mM to 10.0 mM sodium dihydrogen phosphate, and
glycerol at a concentration of 10-30, such as 10, 20, or 30 mg.

In some embodiments, the present disclosure provides the following pharmaceutical compositions:
(1) a pharmaceutical composition comprising about 2.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, and about 5.5 mg/mL phenol, the pH of the pharmaceutical composition being about 7.5 or about 7.4;
(2) a pharmaceutical composition comprising about 4.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, and about 5.5 mg/mL phenol, the pH of the pharmaceutical composition being about 7.5 or about 7.4;
(3) a pharmaceutical composition comprising about 5.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, and about 5.5 mg/mL phenol, the pH of the pharmaceutical composition being about 7.5 or about 7.4;
(4) a pharmaceutical composition comprising about 6.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, and about 5.5 mg/mL phenol, the pH of the pharmaceutical composition being about 7.5 or about 7.4;
(5) a pharmaceutical composition comprising about 8.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, and about 5.5 mg/mL phenol, the pH of the pharmaceutical composition being about 7.5 or about 7.4;
(6) a pharmaceutical composition comprising about 10.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, and about 5.5 mg/mL phenol, the pH of the pharmaceutical composition being about 7.5 or about 7.4;
(7) a pharmaceutical composition comprising about 20.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, and about 5.5 mg/mL phenol, the pH of the pharmaceutical composition being about 7.5 or about 7.4;
(8) a pharmaceutical composition comprising about 2.0 mg/mL, about 4.0 mg/mL, about 5.0 mg/mL, about 6.0 mg/mL, about 8.0 mg/mL, 10 mg/mL, or about 20 mg/mL compound shown as 18# in FIG. 3 or the pharmaceutically acceptable salt thereof, about 5.0 mM sodium dihydrogen phosphate, about 8-9 mg/mL (e.g., about 8.2 mg/mL) sodium chloride, and optionally about 5.5 mg/mL phenol, the pH of the pharmaceutical composition being about 7.5 or about 7.4;
(9) pharmaceutical compositions obtained by replacing 5.0 mM sodium dihydrogen phosphate in the pharmaceutical compositions of (1)-(8) with 10.0 mM sodium dihydrogen phosphate;
(10) pharmaceutical compositions obtained by replacing the osmotic pressure regulator in the pharmaceutical compositions of (1)-(9) with glycerol, the concentration of glycerol being 20 mg; or
(11) the pharmaceutical compositions of (1)-(10) with a final volume of 1 mL, the volume being brought to 1 mL by using water for injection when required.

In some embodiments, the present disclosure provides the following pharmaceutical compositions:
(1) a pharmaceutical composition consisting of: about 2.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, about 5.5 mg/mL phenol, a proper amount of sodium hydroxide and/or a proper amount of hydrochloric acid, and the balance made up of water for injection, the pH of the pharmaceutical composition being about 7.5 or about 7.4;
(2) a pharmaceutical composition consisting of: about 4.0 mg/mL compound shown as 18# in FIG. 3 or the pharmaceutically acceptable salt thereof, 5.0 mM sodium dihydrogen phosphate, about 14.0 mg/mL propylene glycol or about 31.5 mg/mL mannitol, about 5.5 mg/mL phenol, a proper amount of sodium hydroxide and/or a proper amount of hydrochloric acid, and the balance made up of water for injection, the pH of the pharmaceutical composition being about 7.5 or about 7.4;
(3) a pharmaceutical composition consisting of: about 5.0 mg/mL compound shown as 18# in FIG. 3 or the pharmaceutically acceptable salt thereof, 5.0 mM sodium dihydrogen phosphate, about 14.0 mg/mL propylene glycol or about 31.5 mg/mL mannitol, about 5.5 mg/mL phenol, a proper amount of sodium hydroxide and/or a proper amount of hydrochloric acid, and the balance made up of water for injection, the pH of the pharmaceutical composition being about 7.5 or about 7.4;
(4) a pharmaceutical composition consisting of: about 6.0 mg/mL compound shown as 18# in FIG. 3 or the pharmaceutically acceptable salt thereof, 5.0 mM sodium dihydrogen phosphate, about 14.0 mg/mL propylene glycol or about 31.5 mg/mL mannitol, about 5.5 mg/mL phenol, a proper amount of sodium hydroxide and/or a proper amount of hydrochloric acid, and the balance made up of water for injection, the pH of the pharmaceutical composition being about 7.5 or about 7.4;
(5) a pharmaceutical composition consisting of: about 8.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, about 5.5 mg/mL phenol, a proper amount of sodium hydroxide and/or a proper amount of hydrochloric acid, and the balance made up of water for injection, the pH of the pharmaceutical composition being about 7.5 or about 7.4;
(6) a pharmaceutical composition consisting of: 10.0 mg/mL compound shown as 18# in FIG. 3 or the pharmaceutically acceptable salt thereof, 5.0 mM sodium dihydrogen phosphate, about 14.0 mg/mL propylene glycol or 31.5 mg/mL mannitol, 5.5 mg/mL phenol, a proper amount of sodium hydroxide and/or a proper amount of hydrochloric acid, and the balance made up of water for injection, the pH of the pharmaceutical composition being 7.5 or about 7.4;
(7) a pharmaceutical composition consisting of: 20.0 mg/mL compound shown as 18# in FIG. 3 or the pharmaceutically acceptable salt thereof, 5.0 mM sodium dihydrogen phosphate, about 14.0 mg/mL propylene glycol or 31.5 mg/mL mannitol, 5.5 mg/mL phenol, a proper amount of sodium hydroxide and/or a proper amount of hydrochloric acid, and the balance made up of water for injection, the pH of the pharmaceutical composition being 7.5 or about 7.4;
(8) a pharmaceutical composition consisting of: about 2.0 mg/mL, about 4.0 mg/mL, about 5.0 mg/mL, about 6.0 mg/mL, about 8.0 mg/mL, about 10 mg/mL, or about 20 mg/mL compound shown as 18# in FIG. 3 or the pharmaceutically acceptable salt thereof, about 5.0 mM sodium dihydrogen phosphate, 8-9 mg/mL (e.g., about 8.2 mg/mL) sodium chloride, and optionally about 5.5 mg/mL phenol, a proper amount of sodium hydroxide and/or a proper amount of hydrochloric acid, and the balance made up of water for injection, the pH of the pharmaceutical composition being about 7.5 or about 7.4;
(9) pharmaceutical compositions obtained by replacing 5.0 mM sodium dihydrogen phosphate in the pharmaceutical compositions of (1)-(8) with 10.0 mM sodium dihydrogen phosphate;
(10) pharmaceutical compositions obtained by replacing the osmotic pressure regulator in the pharmaceutical compositions of (1)-(9) with glycerol, the concentration of glycerol being 20 mg; or
(11) the pharmaceutical compositions of (1)-(10) with a final volume of 1 mL, the volume being brought to 1 mL by using water for injection when required.

The pharmaceutical composition of the present disclosure already has sufficient stability for being prepared into a drug and can be stable after long-term storage.

In some embodiments, the pharmaceutical composition remains stable at 2-8 °C for at least 3 months, at least 6 months, at least 12 months, at least 18 months, at least 24 months, or at least 36 months. In some embodiments, the pharmaceutical composition remains stable at 25 °C for at least 3 months, at least 6 months, at least 12 months, at least 18 months, or at least 24 months. In some embodiments, the pharmaceutical composition remains stable at 40 °C for at least 7 days, at least 14 days, at least 28 days, at least 1 month, at least 3 months, at least 6 months, at least 12 months, at least 18 months, or at least 24 months.

The present disclosure provides a method for preparing the aforementioned pharmaceutical composition, which comprises the step of dissolving the GLP-1 analog or the pharmaceutically acceptable salt thereof.

The pharmaceutical composition of the present disclosure may be further prepared into a lyophilized formulation for convenience of drug delivery.

The present disclosure provides a lyophilized formulation, wherein the lyophilized formulation is capable of forming the pharmaceutical composition according to any one of the above upon reconstitution.

The present disclosure further provides a method for preparing a lyophilized formulation comprising the GLP-1 analog or the pharmaceutically acceptable salt thereof, which comprises the step of lyophilizing the aforementioned pharmaceutical composition. In alternative embodiments, the lyophilizing comprises the steps of pre-freezing, primary drying, and secondary drying in sequence.

The present disclosure further provides a lyophilized formulation comprising the GLP-1 analog or the pharmaceutically acceptable salt thereof prepared by the aforementioned method for preparing a lyophilized formulation comprising the GLP-1 analog or the pharmaceutically acceptable salt thereof.

The present disclosure further provides a method for preparing a reconstituted solution of the lyophilized formulation comprising the GLP-1 analog or the pharmaceutically acceptable salt thereof, which comprises the step of reconstituting the aforementioned lyophilized formulation, wherein the solution used for the reconstitution is selected from the group consisting of, but is not limited to, water for injection, normal saline, and glucose solution.

The present disclosure further provides a reconstituted solution of the lyophilized formulation comprising the GLP-1 analog or the pharmaceutically acceptable salt thereof prepared by the aforementioned method for preparing a reconstituted solution of the lyophilized formulation comprising the GLP-1 analog or the pharmaceutically acceptable salt thereof.

The present disclosure further provides an article of manufacture or a kit, which comprises a container containing any of the stable pharmaceutical compositions described herein. In some embodiments, the glass bottle is a tubular injection vial made of neutral borosilicate glass.

The present disclosure further provides an article of manufacture, which comprises a container, wherein the container contains the aforementioned pharmaceutical composition, lyophilized formulation, or reconstituted solution of the lyophilized formulation.

The present disclosure further provides the pharmaceutical composition, the lyophilized formulation, or the reconstituted solution of the lyophilized formulation, for use in methods for treating and preventing diseases or disorders.

The present disclosure further provides use of the aforementioned pharmaceutical composition, lyophilized formulation, or reconstituted solution of the lyophilized formulation, in the preparation of a medicament for treating and/or preventing diseases or disorders.

The present disclosure further provides a method for treating and preventing diseases or disorders, which comprises administering to a patient in need thereof a therapeutically effective amount of the aforementioned pharmaceutical composition, lyophilized formulation, or reconstituted solution of the lyophilized formulation.

The present disclosure provides use of the pharmaceutical composition in the preparation of a medicament for treating non-insulin-dependent diabetes, insulin-dependent diabetes, obesity, non-alcoholic fatty liver, hepatic steatosis, diabetic retinopathy, diabetic neuropathy, diabetic nephropathy, insulin resistance, dyslipidemia associated with insulin resistance, and/or dyslipidemia associated with diabetes.

The present disclosure provides a pharmaceutical composition for use in treating non-insulin-dependent diabetes/type II diabetes, insulin-dependent diabetes, obesity, non-alcoholic fatty liver, hepatic steatosis, dyslipidemia associated with insulin resistance, and/or dyslipidemia associated with diabetes.

The present disclosure provides a method for treating non-insulin-dependent diabetes/type II diabetes, insulin-dependent diabetes, obesity, non-alcoholic fatty liver, hepatic steatosis, dyslipidemia associated with insulin resistance, and/or dyslipidemia associated with diabetes, which comprises administering to a subject in need thereof the pharmaceutical composition of the present disclosure.

The present disclosure provides a compound capable of simultaneously activating a GLP-1 receptor and a GIP receptor, and in some embodiments, the GLP-1 analog has greater agonist activity for the GLP-1R than for the GIP receptor.

In some specific embodiments, the GLP-1 analog of the present disclosure has a ratio of the agonist activity for the GLP-1R to the agonist activity for the GIP receptor of (1-10):1, (1.1-10):1, (1.1-9.5):1, (1.1-9):1, (1.1-8.5):1, (1.1-8):1, (1.1-7.5):1, (1.1-7):1, (1.1-6.5):1, (1.1-6):1, (1.2-10):1, (1.2-9.5):1, (1.2-9):1, (1.2-8.5):1, (1.2-8):1, (1.2-7.5):1, (1.2-7):1, (1.2-6.5):1, (1.2-6):1, (1.3-10):1, (1.3-9.5):1, (1.3-9):1, (1.3-8.5):1, (1.3-8):1, (1.3-7.5):1, (1.3-7):1, (1.3-6.5):1, (1.3-6):1, (1.4-10):1, (1.4-9.5):1, (1.4-9):1, (1.4-8.5):1, (1.4-8):1, (1.4-7.5):1, (1.4-7):1, (1.4-6.5):1, (1.4-6):1, (1.5-10):1, (1.5-9.5):1, (1.5-9):1, (1.5-8.5):1, (1.5-8):1, (1.5-7.5):1, (1.5-7):1, (1.5-6.5):1, (1.5-6):1, (2-10):1, (2-9.5):1, (2-9):1, (2-8.5):1, (2-8):1, (2-7.5):1, (2-7):1, (2-6.5):1, (2-6):1, (2.5-10):1, (2.5-9.5):1, (2.5-9):1, (2.5-8.5):1, (2.5-8):1, (2.5-7.5):1, (2.5-7):1, (2.5-6.5):1, (2.5-6):1, (3-10):1, (3-9.5):1, (3-9):1, (3-8.5):1, (3-8):1, (3-7.5):1, (3-7):1, (3-6.5):1, (3-6):1, (3.5-10):1, (3.5-9.5):1, (3.5-9):1, (3.5-8.5):1, (3.5-8):1, (3.5-7.5):1, (3.5-7):1, (3.5-6.5):1, (3.5-6):1, (4-10):1, (4-9.5):1, (4-9):1, (4-8.5):1, (4-8):1, (4-7.5):1, (4-7):1, (4-6.5):1, (4-6):1, (4.5-10):1, (4.5-9.5):1, (4.5-9):1, (4.5-8.5):1, (4.5-8):1, (4.5-7.5):1, (4.5-7):1, (4.5-6.5):1, (4.5-6):1, (5-10):1, (5-9.5):1, (5-9):1, (5-8.5):1, (5-8):1, (5-7.5):1, (5-7):1, (5-6.5):1, (5-6):1, (5-5.5):1, (5.1-5.5):1, (5.2-5.4):1, (5.2-5.3):1, or any range or point of value therebetween, e.g., about 1:1, about 1.1:1, about 1.2:1, about 1.3:1, about 1.4:1, about 1.5:1, about 2:1, about 2.5:1, about 3:1, about 3.5:1, about 4:1, about 4.5:1, about 5:1, about 5.2:1, about 5.3:1, about 5.4:1, about 5.5:1, about 6:1, about 6.5:1, about 7:1, about 7.5:1, about 8:1, about 8.5:1, about 9:1, about 9.5:1, or about 10:1. The above ratio is a normalized ratio of data from *in vitro* assay of corresponding agonist activity. For example, the corresponding agonist activity can be determined by a cAMP-Gs kinetic kit. In this context, the expression (1-10):1 and the expression 1:1 to 10:1 have the same meaning.

In another embodiment, the present disclosure provides the above GLP-1 analog and the pharmaceutically acceptable salt thereof. The GLP-1 analog provided by the present disclosure is an amphoteric compound that can exhibit both acidity and basicity. The GLP-1 analog provided by the present disclosure can be reacted with acidic or basic compounds to form salts by those skilled in the art using well known techniques.

The pharmaceutical composition comprising the GLP-1 analog according to the present disclosure can be used for treating patients in need of such treatment by parenteral administration. For the parenteral routes of administration, subcutaneous injection, intramuscular injection, or intravenous injection may be selected. The polypeptide dual-agonist compound of the present disclosure may also be administered by the transdermal route, optionally via an iontophoretic patch; or by the transmucosal route.

The GLP-1 analog provided by the present disclosure is synthesized by a solid-phase synthesis method. As an example, the synthetic vector is Rink-amide MBHA (Xi'an sunresin Tech Ltd.) resin. During the synthesis, the α-amino of the amino acid derivative used is protected by the Fmoc (fluorenylmethoxycarbonyl) group. As an example, for the side chain of an amino acid, the following protecting groups are selected according to the difference of functional groups: the sulfhydryl of the cysteine side chain, the amino of the asparagine and glutamine side chains, and the imidazolyl of the histidine side chain are protected by Trt (trityl); the guanidyl of the arginine side chain is protected by Pbf (2,2,4,6,7-pentamethyldihydrobenzofuran-5-sulfonyl); the indolyl of the tryptophan side chain and the amino of the lysine side chain are protected by Boc (*tert*-butyloxycarbonyl); and the carboxyl of the aspartic acid and glutamic acid side chains, the hydroxyl of the threonine side chain, the phenolic group of the tyrosine side chain, and the hydroxyl of the serine side chain are protected by *t*-Bu (*tert*-butyl). As an example, during the synthesis, the carboxyl of the C-terminal amino acid residue of the polypeptide is firstly condensed to the insoluble Rink-amide MBHA polymer resin in the form of an amide bond; then the Fmoc protecting group on the α-amino is removed using an *N*,*N*-dimethylformamide (DMF) solution containing 20% 4-methylpiperidine; and then the solid-phase support is condensed in excess with the next amino acid derivative in the polypeptide sequence to form an amide bond to extend the peptide chain. The procedures of "condensation → washing → deprotection → washing → the next round of amino acid condensation" were repeated to enable the desired length of the polypeptide chain to be synthesized; finally, a mixed solution of trifluoroacetic acid:water:triisopropylsilane (as an example, 90:5:5, v:v:v) is reacted with the resin to cleave the polypeptide from the solid-phase support, and the polypeptide is precipitated using frozen methyl *tert*-butyl ether (5 volume) to obtain a crude solid product of the GLP-1 analog. The crude solid product of the polypeptide is dissolved in an acetonitrile/water mixed solution containing 0.1% trifluoroacetic acid, and purified and separated using a C-18 reversed-phase preparative chromatographic column to obtain a pure product of the GLP-1 analog.

According to some embodiments, the present disclosure further provides a kit-of-parts, which comprises:
- the GLP-1 analog or the pharmaceutically acceptable salt thereof according to the present disclosure; and
- an additional therapeutic agent selected from any one of or a combination of: an anti-obesity agent, an antidiabetic agent, an antihypertensive agent, and a lipid-lowering agent; wherein the GLP-1 analog or the pharmaceutically acceptable salt thereof and the additional therapeutic agent are each placed in a separate container. In some embodiments, the GLP-1 analog or the pharmaceutically acceptable salt thereof and the additional therapeutic agent are administered to a subject separately or in combination (e.g., simultaneously or sequentially).

In certain embodiments, the pharmaceutical composition of the present disclosure and an administration device (e.g., a syringe, an injection pen, or an automatic syringe) are provided in combination. As an example, the pharmaceutical composition of the present disclosure is pre-filled in the administration device for self-administration by a subject at home. As another example, the pharmaceutical composition of the present disclosure and the administration device are provided separately.

### BRIEF DESCRIPTION OF THE DRAWINGS

FIG. 1 shows the effect of the compound of the present disclosure on the rate of change in body weight of diet-induced-obesity mice.
FIG. 2 shows the effect of the compound of the present disclosure on daily food intake ration of diet-induced-obesity mice.
FIG. 3 shows the structures of exemplary compounds of the present disclosure.

### DETAILED DESCRIPTION

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains.

The amino acid sequences of the present disclosure contain the standard single-letter or three-letter codes for twenty amino acids, and all amino acid residues in the present disclosure are preferably in the L-configuration unless specifically stated. In addition, Aib refers to α-aminoisobutyric acid, D-Ala refers to D-alanine, Orn refers to ornithine, Dap refers to 2,3-diaminopropionic acid, and Dab refers to 2,4-diaminobutyric acid.

The term "agonist" is defined as a substance having an activating effect on the GLP-1 receptor or on the GIP receptor.

The term "GLP-1/GIP dual-agonist" as used in the context of the present disclosure refers to a substance or ligand that can activate the GLP-1 receptor and the GIP receptor. In the present disclosure, the term "treat", "treating", or "treatment" includes inhibiting, alleviating, stopping, or reversing the progression or severity of an existing symptom or condition.

The term "natural amino acids" refers to 20 conventional amino acids, i.e., alanine (A), cysteine (C), aspartic acid (D), glutamic acid (E), phenylalanine (F), glycine (G), histidine (H), isoleucine (I), lysine (K), leucine (L), methionine (M), asparagine (N), proline (P), glutamine (Q), arginine (R), serine (S), threonine (T), valine (V), tryptophan (W), and tyrosine (Y).

The term "unnatural amino acids" refers to amino acids that are not naturally encoded or are not found in the genetic code of any organism. For example, the unnatural amino acids may be completely synthetic compounds. Examples of unnatural amino acids include, but are not limited to, hydroxyproline, γ-carboxyglutamic acid, O-phosphoserine, azetidinecarboxylic acid, 2-aminoadipic acid, 3-aminoadipic acid, β-alanine, aminopropionic acid, 2-aminobutyric acid, 4-aminobutyric acid, 6-aminohexanoic acid, 2-aminoheptanoic acid, 2-aminoisobutyric acid, 3-aminoisobutyric acid, 2-aminopimelic acid, *tert*-butylglycine, 2,4-diaminoisobutyric acid (Dap), desmosine, 2,2'-diaminopimelic acid, 2,3-diaminopropionic acid (Dab), *N*-ethylglycine, *N*-methylglycine, *N*-ethylasparagine, homoproline, hydroxylysine, allo-hydroxylysine, 3-hydroxyproline, 4-hydroxyproline, isodesmosine, allo-isoleucine, *N*-methylalanine, *N*-methylglycine, *N*-methylisoleucine, *N*-methylpentylglycine, *N*-methylvaline, naphthalanine, norvaline, norleucine, ornithine (Orn), D-omithine, D-arginine, *p*-aminophenylalanine, pentylglycine, pipecolic acid, and thioproline. In addition, the term also includes derivatives obtained by chemical modification of the C-terminal carboxyl (or N-terminal amino and/or side chain functional group) of a natural amino acid (or unnatural amino acid).

The term "alkyl" refers to a saturated aliphatic hydrocarbon group which is a linear or branched group containing 1 to 20 carbon atoms, e.g., alkyl containing 1 to 8 carbon atoms, e.g., alkyl containing 1 to 6 carbon atoms, e.g., alkyl containing 1 to 3 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof, and the like. Alkyl may be, for example, a lower alkyl containing 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. Alkyl may be substituted or unsubstituted. When it is substituted, the substituent may be substituted at any accessible point of attachment, and the substituent may be one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, and a carboxylate group. The substituted alkyl of the present disclosure may be methyl, ethyl, isopropyl, tert-butyl, haloalkyl, deuterated alkyl, alkoxy-substituted alkyl, or hydroxy-substituted alkyl.

The expressions "X is selected from the group consisting of A, B or C", "X is selected from the group consisting of A, B and C", "X is A, B or C", "X is A, B and C", and the like all carry the same meaning, i.e., X may be any one or more of A, B and C.

The "modification" of the amino acid as described in the present disclosure refers to substitution, addition, or deletion of an amino acid, including substitution or addition of any one or more of the 20 natural amino acids.

The term "natural GLP-1" refers to a naturally occurring molecule of the glucagon or exendin family of peptides, wherein the glucagon family of peptides is encoded by the pre-proglucagon gene and includes three small peptides with high homology, i.e., glucagon (1-29), GLP-1 (1-37), and GLP-2 (1-33); and exendins are peptides expressed in lizards and, like GLP-1, are insulinotropic. In some embodiments, the term "natural GLP-1" also refers to human GLP-1 (7-37) and human GLP-1 (7-36).

The term "GLP-1 analog" refers to a substance having up to 25, up to 24, up to 23, up to 22, up to 21, up to 20, up to 19, up to 18, up to 17, up to 16, up to 15, up to 14, up to 13, up to 12, up to 11, up to 10, up to 9, up to 8, up to 7, up to 6, up to 5, up to 4, up to 3, up to 2, or 1 amino acid modification or chemical modification compared with natural GLP-1 (in particular with human GLP-1 (7-37) and human GLP-1 (7-36)), wherein the amino acid modification may be an amino acid substitution, addition, and/or deletion, and the chemical modification may be a chemical modification with a group selected from the group consisting of the following groups: amide, carbohydrate, alkyl, acyl, ester, a polyethylene glycol (PEG) group, a sialylation group, a glycosylation group, and the like.

The term amino acid "substitution" as described in the present disclosure refers to the substitution of one amino acid residue with a different amino acid residue.

The term "polyethylene glycol" or "PEG" refers to a mixture of polycondensates of ethylene oxide and water and is present in a linear or branched form and represented by the general formula H(OCH₂CH₂)ₙOH, where n is at least equal to 9. Unless further stated, this term includes polymers of polyethylene glycol having an average total molecular weight selected from the group consisting of 5,000 to 40,000 daltons.

The term "fatty acid" refers to carboxylic acid with an aliphatic long tail (chain) and may be saturated or unsaturated. The fatty acids in the present disclosure are carboxylic acids having a C4-C30 linear or branched aliphatic group.

The term "peptide" as used in the present disclosure encompasses peptides having modified amino and carboxyl termini. For example, an amino acid chain containing a terminal carboxylic acid substituted with an amide group is also included within the amino acid sequence designated as a natural amino acid.

All of the hydrogen atoms described in the present disclosure may be substituted with their isotopes (protium, deuterium, and tritium), and any hydrogen atom in the compound of the present disclosure to which the present disclosure relates may also be substituted with an isotope atom.

"Optional" or "optionally" means that the event or circumstance subsequently described may, but does not necessarily, occur and that the description includes instances where the event or circumstance occurs or does not occur. For example, "a heterocyclyl group optionally substituted with alkyl" means that the alkyl may, but does not necessarily, exist and that the description includes instances where the heterocyclyl group is or is not substituted with the alkyl.

The term "substituted" means that one or more, preferably up to 5, more preferably 1 to 3 hydrogen atoms in the group are independently substituted with a substituent. A substituent is only in its possible chemical position, and those skilled in the art will be able to determine (experimentally or theoretically) possible or impossible substitution without undue efforts. For example, it may be unstable when amino or hydroxy having a free hydrogen is bound to a carbon atom having an unsaturated (e.g., olefinic) bond.

"Pharmaceutical composition" refers to a mixture containing one or more of the compounds described herein or physiologically/pharmaceutically acceptable salts or prodrugs thereof, and other chemical components, wherein the other components are, for example, physiologically/pharmaceutically acceptable carriers and excipients. The pharmaceutical composition is intended to promote administration to an organism and facilitate the absorption of the active ingredient so that it can exert its biological activity. "Agonist activity" refers to the ability of the compound according to the present disclosure to activate the human GIP receptor and the human GLP-1 receptor. In some examples, "agonist activity" is embodied in a relatively active form and specifically refers to the ratio of the activation ability of the compound of the present disclosure against GLP-1R to that against the GIP receptor.

"Pharmaceutically acceptable salt" refers to the salts of the compound of the present disclosure, which are safe and effective for use in the body of a mammal and possess the requisite biological activities. Generally, such a salt is an acid addition or base addition salt. Acid addition salts include inorganic acid salts and organic acid salts.

Semaglutide refers to a once-a-week GLP-1 receptor single agonist polypeptide drug developed by Novo Nordisk in Denmark, which is currently approved and marketed in the United States, Japan, and the European Union.

LY3298176 refers to a once-a-week GIP receptor/GLP-1 receptor dual-agonist polypeptide drug developed by Eli Lilly, which is currently in phase III clinical trials in several countries. The structure is as follows:

YAibEGTFTSDYSIAibLDKIAQ**K**AFVQWLIAGGPSSGAPPPS-NH₂, wherein the K at position 20 is modified with a fatty acid shown below as

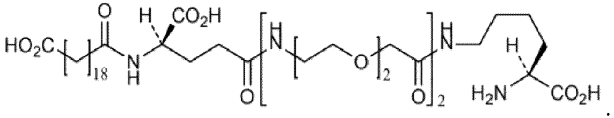

"Buffer" refers to a buffer that resists changes in pH by the action of its acid-base conjugate components. Examples of buffers that keep the pH within an appropriate range include acetate, succinate, citrate, phosphate, gluconate, histidine salt, oxalate, lactate, phosphate, citrate, tartrate, fumarate, glycylglycine, and other organic acid buffers.

"Histidine salt buffer" is a buffer comprising histidine ions. Examples of histidine salt buffers include histidine-hydrochloride buffers, histidine-acetate buffers, histidine-phosphate buffers, histidine-sulfate buffers, and the like, e.g., histidine-acetate buffers or histidine-hydrochloride buffers. Histidine-acetate buffers are prepared from histidine and acetic acid, and histidine salt buffers are prepared from histidine and hydrochloric acid.

"Citrate buffer" is a buffer comprising citrate ions. Examples of citrate buffers include citric acid-sodium citrate, citric acid-potassium citrate, citric acid-calcium citrate, citric acid-magnesium citrate, and the like. The citrate buffer may be citric acid-sodium citrate.

"Succinate buffer" is a buffer comprising succinate ions. Examples of succinate buffers include succinic acid-sodium succinate, succinic acid-potassium succinate, succinic acid-calcium succinate, and the like. The succinate buffer may be succinic acid-sodium succinate.

"Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, and the like. The phosphate buffer may be disodium hydrogen phosphate-sodium dihydrogen phosphate.

"Acetate buffer" is a buffer comprising acetate ions. Examples of acetate buffers include acetic acid-sodium acetate, acetic acid-histidine salt, acetic acid-potassium acetate, acetic acid-calcium acetate, acetic acid-magnesium acetate, and the like. The acetate buffer may be acetic acid-sodium acetate.

"Pharmaceutical composition" refers to a mixture containing one or more of the compounds described herein or physiologically/pharmaceutically acceptable salts or prodrugs thereof, and other chemical components, wherein the other components are, for example, physiologically/pharmaceutically acceptable carriers and excipients. The pharmaceutical composition is intended to promote administration to an organism and facilitate the absorption of the active ingredient so that it can exert its biological activity. As used herein, "pharmaceutical composition" and "formulation" may be used interchangeably.

Unless otherwise stated, the solvent in the pharmaceutical composition described in the present disclosure in solution form is water.

"Lyophilized formulation" refers to a formulation or a pharmaceutical composition obtained by lyophilizing a pharmaceutical composition or formulation in liquid or solution form *in vacuum.*

The term "about" or "approximately" as used herein means that a numerical value is within an acceptable error range for the particular value determined by one of ordinary skill in the art, and the numerical value depends in part on how the value is measured or determined (i.e., the limits of the measurement system). For example, "about" may mean a standard deviation within 1 or more than 1 in each practice in the art. Or, "about" or "substantially comprise" may mean a range of up to ±20%; for example, a pH of about 5.5 means a pH of 5.5±1.1. Furthermore, particularly for biological systems or processes, the term may mean up to an order of magnitude or up to 5-fold of a numerical value. Unless otherwise stated, when a specific value is provided in the present application and claims, the meaning of "about" or "substantially comprise" should be assumed to be within an acceptable error range for that specific value.

The pharmaceutical composition described in the present disclosure can achieve the effect of being stable: the GLP-1 analog in the pharmaceutical composition substantially retains its physical and/or chemical stability and/or biological activity after storage; for example, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

A stable pharmaceutical formulation is one in which no significant change is observed under the following conditions: storage at refrigeration temperature (2-8 °C) for at least 3 months, at least 6 months, at least 1 year, at least 2 years, or at most 2 years. In addition, stable liquid formulations include liquid formulations that exhibit desirable features after storage at 25 °C for periods including 1 month, 3 months, and 6 months, or storage at 40 °C for periods including 1 month. Typical acceptable criteria for stability are as follows: typically, no more than about 10%, for example, no more than about 5%, of the GLP-1 analog monomer is degraded as measured by SEC-HPLC. The pharmaceutical formulation is colorless, or clear to slightly opalescent, by visual analysis. The concentration, pH, and osmolality of the formulation have a change of no more than ±10%. Typically, clippings of no more than about 10%, for example, no more than about 5%, are observed. Typically, aggregation of no more than about 10%, for example, no more than about 5%, is formed.

A GLP-1 analog "retains its physical stability" in a pharmaceutical formulation if it shows no significant increase in aggregation, precipitation, and/or denaturation upon visual inspection of color and/or clarity, or as determined by UV light scattering, size exclusion chromatography (SEC), and dynamic light scattering (DLS). Changes in protein conformation can be assessed by fluorescence spectroscopy (which determines the protein tertiary structure) and by FTIR spectroscopy (which determines the protein secondary structure).

A GLP-1 analog "retains its chemical stability" in a pharmaceutical formulation if it shows no significant chemical change. Chemical stability can be evaluated by detecting and quantifying chemically changed protein. Degradation processes that often change the chemical structure of proteins include hydrolysis or clipping (assessed by methods such as size exclusion chromatography and SDS-PAGE), oxidation (assessed by methods such as peptide mapping in combination with mass spectroscopy or MALDI/TOF/MS), deamidation (assessed by methods such as ion-exchange chromatography, capillary isoelectric focusing, peptide mapping, and isoaspartic acid determination), and isomerization (assessed by isoaspartic acid content determination, peptide mapping, etc.).

A GLP-1 analog "retains its biological activity" in a pharmaceutical formulation if its biological activity at a given time is within a predetermined range of the biological activity exhibited during the preparation of the pharmaceutical formulation. The biological activity of a GLP-1 analog can be determined, for example, by an antigen binding assay.

The three-letter and single-letter codes for amino acids used in the present disclosure are as described in *J. biol. chem,* 243, p3558 (1968).

### Examples

The following specific embodiments are provided herein only for illustrating the present disclosure in more detail, rather than limiting the present disclosure. Experimental procedures without specific conditions indicated in the examples of the present disclosure are generally conducted according to conventional conditions or according to conditions recommended by the manufacturers of the starting materials or commercial products. Reagents without specific sources indicated are commercially available conventional reagents.

**Table 1. Part of experimental reagents and sources**

| Reagent | Source |
|---|---|
| Rink-amide MBHA resin | Xi'an sunresin Tech Ltd. |
| HCTU (O-(6-chloro-1-benzotriazol-1-yl)-*N*,*N*,*N'*,*N'*-tetramethyluronium hexafluorophosphate) | Highfine Tech Ltd., Sunzhou |
| Fmoc-Aib-OH | GL Biochem |
| Fmoc-L-Lys(Mtt)-OH | GL Biochem |
| *N*,*N*-dimethylformamide | SinoPharm |
| Dichloromethane | SinoPharm |
| Trifluoroacetic acid | SinoPharm |
| Triisopropylsilane | Sigma-Aldrich |
| Hexafluoroisopropanol | Sigma-Aldrich |
| Acetonitrile | Merck-Millipore |
| Diisopropylethylamine | Sigma-Aldrich |
| 4-methylpiperidine | TCI Chemicals |
| Methyl *tert*-butyl ether | TCI Chemicals |
| Boc-L-Tyr(tBu)-OH | GL Biochem |
| Fmoc-NH-PEG₂-COOH | GL Biochem |
| Fmoc-L-Glu-OtBu | GL Biochem |
| HOOC-(CH₂)₁₈-COOtBu | ChinaPeptides Co., Ltd, Suzhou |
| 4-methylmorpholine | TCI Chemicals |
| DMEM/F12 | Gibco 11330032 |
| Casein | Sigma C3400-500G |
| 3-Isobutyl-1-methylxanthine | Sigma 17018-250 MG |
| cAMP - Gs Dynamic kit - 20,000 tests | Cisbio 62AM4PEC |
| Corning^{®} 384 well microplate, low volume | Sigma CLS4514-50EA |
| 96-well V-bottom plate (PS) | Axygen WIPP02280 |
| Countess^{®} Cell Counting Chamber Slides | Invitrogen C10228 |
| puromycin | ThermoFisher A1113803 |
| Hygromycin B | Sigma A1720 |
| PBS | Gibco 10010023 |
| 0.25%Trypsin-EDTA(1X), Phenol Red | ThermoFisher 25200-114 |
| Gibco^{™} Fetal Bovine Serum, Qualified, Australia Origin | ThermoFisher 10099-141 |
| Glucose | Sigma G8270-100G |

**Table 2. Part of experimental instruments and sources**

| Instrument | Source |
|---|---|
| H-CLASS analytical ultra-high performance liquid chromatograph | WATERS |
| Agilent 1290-6530 ultra-high performance liquid chromatograph/mass spectrometer combination | Agilent |
| Labconco multifunctional freeze dryer | Thermo-Fisher Scientific |
| Prep150 preparative high performance liquid chromatograph | WATERS |
| Prelude-X automatic polypeptide synthesizer | Protein Technology Inc |
| Multichannel high-speed centrifuge | Sigma |
| Refrigerated centrifuge 5810R | Eppendorf 5810R |
| Active glucometer | Roche |
| Microplate reader | BioTek H1MFD |

### Example 1. Chemical Synthesis of Compound 18#

### 1. Synthesis of polypeptide skeleton

Rink-amide MBHA resin (degree of substitution: 0.48 mMole/g, 0.1 mMol) was taken and placed in a polypropylene reaction tube for solid phase synthesis of the polypeptide; N,N-dimethylformamide (DMF, 10 mL) was added to swell the resin for 10 min under nitrogen-blowing; DMF was removed *in vacuum,* and fresh DMF (10 mL) was added to wash the resin; after repeated washing of the resin twice, the solid phase synthesis of the polypeptide was performed on a Prelude-X automatic polypeptide synthesizer using Fmoc/tBu strategy, in which 10 equivalents of amino acid residues activated by HCTU and 4-methylmorpholine (molar ratio of HCTU to 4-methylmorpholine to amino acid residues was 1:2:1) were reacted in DMF at room temperature for 25 min for amide bond condensation, so as to achieve coupling. Deprotection of the N-terminal Fmoc protecting group was performed by 2 reactions (10 min each) at room temperature using a DMF solution containing 20% 4-methylpiperidine. In the synthesis of a polypeptide skeleton, the N-terminal amino acid residue was constructed using Boc-L-Tyr (tBu)-OH and subjected to secondary condensation, which was necessary for improving the quality of a crude peptide.

### 2. Selective deprotection of resin-peptide protecting group Mtt and fatty acid modification of side chain

After the extension of the polypeptide skeleton (or called resin-peptide) was completed, a mixed solution (10 mL) of dichloromethane containing 30% hexafluoroisopropanol was added, and the mixture was shaken at room temperature for 45 min, and then the mixed solution was removed *in vacuum;* a mixed solution (10 mL) of dichloromethane containing 30% hexafluoroisopropanol was added, and the mixture was shaken at room temperature for 45 min, and then the mixed solution was removed *in vacuum.* After the reaction was completed, the resin was washed 6 times with DMF. The lysine side chain at position 14 was extended using a Prelude-X automatic polypeptide synthesizer, with an additional coupling/deprotection cycle involving the amino acid components Fmoc-NH-PEG₂-COOH and Fmoc-L-Glu-OtBu. All couplings were performed in DMF at room temperature for 25 min using 10 equivalents of amino acid residues activated by HCTU and 4-methylmorpholine (molar ratio of HCTU to 4-methylmorpholine to amino acid residues was 1:2:1). Deprotection of the N-terminal Fmoc protecting group was performed by 2 reactions (10 min each) at room temperature using a DMF solution containing 20% 4-methylpiperidine. After the finally obtained resin was washed three times with DCM and DMF separately, a mixed solution (8 mL) of DMF containing 10 equivalents of HOOC-(CH₂)₁₈-COOtBu, 10 equivalents of HCTU, and 20 equivalents of diisopropylethylamine (DIEA) was added, and the mixture was reacted at room temperature for 4 h to complete the fatty acid modification of the side chain.

### 3. Product cleavage

The resin-peptide obtained in the previous step was washed 3 times with DMF and DCM sequentially and dried in vacuum, followed by the addition of a freshly prepared cleavage buffer (trifluoroacetic acid:triisopropylsilane:water = 90:5:5, v:v:v), and the mixture was shaken at room temperature for 3-4 h. After the reaction was completed, the mixture was filtered and the resin was washed twice with trifluoroacetic acid. The filtrates were combined before a large amount of frozen methyl tert-butyl ether was added to precipitate a solid. The mixture was centrifuged and the supernatant was discarded to obtain a crude polypeptide of **compound 18#.**

### 4. Purification by reversed-phase liquid chromatography

The crude polypeptide of **compound 18#** was dissolved in a mixed solvent containing 0.1% trifluoroacetic acid, 20% acetonitrile, and 20% acetic acid/water, and the solution was filtered through a 0.22 µm membrane; the filtrate was separated using a WATERS Prep150 LC reversed-phase high performance liquid chromatography system with buffers A (0.1% trifluoroacetic acid, 10% acetonitrile, and water) and B (0.1% trifluoroacetic acid, 90% acetonitrile, and water). The chromatographic column was an X-SELECT OBD C-18 reversed-phase chromatographic column, and in the purification process, the detection wavelength of the chromatograph was set as 220 nm, and the flow rate was 15 mL/min. The related fractions of the product were collected and lyophilized to obtain a pure polypeptide product of compound 1#, with the yield of 18%. The purity of the pure polypeptide product was determined by a combination of analytical high performance liquid chromatography and ultra-high performance liquid chromatography/mass spectrometry, with the purity of 92.81%. The molecular structure of compound 18# is:
H-YAibEGTFTSDYSIYK(OEG-OEG-yGlu-C20-OH)EKIAAQEFVNWLLAGGPSSG APPPS-NH₂, with the structural formula shown as the structure of 18# in FIG. 3.

### Example 2. Chemical Synthesis of Other Compounds

The compounds in Table 3 were synthesized using the experimental protocol of Example **1**.

**Table 3. Compounds of the present disclosure**

| Compound No. and molecular structure thereof | |
|---|---|
| 1# | H-YAibEGTFTSDYSIYK(OEG-OEG-yGlu-C20-OH)DKIAAQEFVNWLIAGGPSSGAPPPS-NH₂ |
| 2# | H-YAibEGTFTSDYSIYK(OEG-OEG-yGlu-C20-OH)DRIAAQEFVNWLIAGGPSSGAPPPS-NH₂ |
| 3# | H-YAibEGTFTSDYSIYK(OEG-OEG-yGlu-C20-OH)DKIAAQEFINWLIAGGPSSGAPPPS-NH₂ |
| 4# | H-YAibEGTFTSDYSIYK(OEG-OEG-yGlu-C20-OH)DRIAAQEFINWLIAGGPSSGAPPPS-NH₂ |
| 5# | H-YAibEGTFTSDYSIYK(OEG-OEG-yGlu-C20-OH)DKIAAQEFINWLLAGGPSSGAPPPS-NH₂ |
| 6# | H-YAibEGTFTSDYSIYK(OEG-OEG-yGlu-C20-OH)DRIAAQEFVNWLLAGGPSSGAPPPS-NH₂ |
| 7# | H-YAibEGTFTSDYSIYK(OEG-OEG-yGlu-C20-OH)DKIAAQEFVNWLLAGGPSSGAPPPS-NH₂ |
| 8# | H-YAibEGTFTSDYSIYLEK(OEG-OEG-yGlu-C20-OH)IAAQEFVNWLLAGGPSSGAPPPS-NH₂ |
| 9# | H-YAibEGTFTSDYSIYLEK(OEG-OEG-yGlu-C20-OH)IAAQEFVNWLIAGGPSSGAPPPS-NH₂ |
| 10# | H-YAibEGTFTSDYSIYLEK(OEG-OEG-yGlu-C20-OH)IAAQEFINWLIAGGPSSGAPPPS-NH₂ |
| 11# | H-YAibEGTFTSDYSIYLEK(OEG-OEG-yGlu-C20-OH)IAAQEFINWLLAGGPSSGAPPPS-NH₂ |
| 12# | H-YAibEGTFTSDYSIYK(OEG-OEG-yGlu-C20-OH)EKIAAQEFVNWLIAGGPSSGAPPPS-NH₂ |
| 13# | H-YAibEGTFTSDYSIYK(OEG-OEG-yGlu-C20-OH)ERIAAQEFVNWLIAGGPSSGAPPPS-NH₂ |
| 14# | H-YAibEGTFTSDYSIYK(OEG-OEG-yGlu-C20-OH)EKIAAQEFINWLIAGGPSSGAPPPS-NH₂ |
| 15# | H-YAibEGTFTSDYSIYK(OEG-OEG-yGlu-C20-OH)ERIAAQEFINWLIAGGPSSGAPPPS-NH₂ |
| 16# | H-YAibEGTFTSDYSIYK(OEG-OEG-yGlu-C20-OH)EKIAAQEFINWLLAGGPSSGAPPPS-NH₂ |
| 17# | H-YAibEGTFTSDYSIYK(OEG-OEG-yGlu-C20-OH)ERIAAQEFVNWLLAGGPSSGAPPPS-NH₂ |

The purity of the compounds was determined by a combination of analytical high performance liquid chromatography and ultra-high performance liquid chromatography/mass spectrometry, with the purity of some of the compounds shown in Table 4 below.

**Table 4. Purity and molecular weight of compounds 8# to 11# determined by combination of analytical high performance liquid chromatography and liquid chromatography/mass spectrometry**

| Compound No. | Purity |
|---|---|
| 8# | 96.30% |
| 9# | 93.28% |
| 10# | 94.56% |
| 11# | 92.18% |

### Biological Evaluation

The present disclosure is further described and explained below with reference to test examples, but these examples are not intended to limit the scope of the present disclosure.

### Example 3. Evaluation of Agonist Activity of Compounds of the Present Disclosure Against Glucagon-Like Peptide-1 Receptor (GLP-1R)

### 1. Experimental objective:

This test example was intended to determine agonist activity of the compounds of the present disclosure against the glucagon-like peptide-1 receptor (GLP-1R).

### 2. Experimental procedures:

Cryopreserved CHO-K1/GLP-1R/CRE-luc stable cell strains (which can be prepared by conventional methods in the art) were taken out of a liquid nitrogen tank, rapidly thawed in a water bath at 37 °C, resuspended in a DMEM/F12 medium, and centrifuged, and the cells were washed once, resuspended in an assay buffer, i.e., DMEM/F12 medium containing 0.1% casein, subjected to the adjustment of cell density with the assay buffer, and seeded in a 384-well plate (Sigma Cat# CLS4514) at a density of 2500 cells/5 µL/well. Then 2.5 µL of an IBMX working solution (Sigma Cat# I7018) prepared in a buffer (the final concentration of IBMX was 0.5 mM) and 2.5 µL of polypeptide samples diluted in a gradient were added to each well, and the plate was centrifuged at 1000 rpm for 1 min, shaken for 30 s for mixing well, and left to stand for incubation at room temperature for 30 min. Detection was performed using the Cisbio cAMP-Gs Dynamic kit (Cisbio Cat# 62AM4PEC), and cAMP-d2 and Anti-cAMP-Eu³⁺-Cryptate were each subjected to a 20-fold dilution using cAMP Lysis & Detection Buffer and each mixed well. 5 µL of diluted cAMP-d2 solution was added to each well, followed by the addition of 5 µL of diluted Anti-cAMP-Eu³⁺-Cryptate solution, and the mixture was shaken for 30 s for mixing well and then incubated at room temperature for 1 h in the dark.

### 3. Data processing:

HTRF signal reading was performed using a Biotek Synergy H1 microplate reader at an excitation wavelength of 320 nm and emission wavelengths of 620 nm and 665 nm. The signal ratios (665 nm/620 nm*10,000) were calculated and fitted non-linearly to sample concentrations in GraphPad Prism 6 using a four-parameter equation to obtain EC₅₀ values, with the specific data shown in Table 5 below.

### Example 4. Evaluation of Agonist Activity of Compounds of the Present Disclosure Against Glucose-dependent Insulinotropic Polypeptide Receptor (GIPR)

### 1. Experimental objective:

This example was intended to determine agonist activity of the compounds of the present disclosure against the glucose-dependent insulinotropic polypeptide receptor (GIPR).

### 2. Experimental procedures:

Wild-type CHO-K1 cells were collected, and the cell suspension was adjusted to an appropriate density, seeded in a 6-well plate at 2 mL/well, and placed in an incubator at 37 °C with 5% CO₂ for adherence culture overnight. The transfection mixture (hGIP receptor plasmid, Fugene HD (Promega Cat# E2311), and OptiMEM (Gibco Cat# 31985070)) was mixed well, left to stand at room temperature for 15 min, and added to the corresponding cell wells in a volume of 100 µL, followed by transfection for 24 h to enable the overexpression of the hGIP receptor on the surface of CHO-K1 cells. After the transient transfection was completed, the cells in the 6-well plate were collected, washed once with an assay buffer, i.e., DMEM/F12 medium (Gibco Cat# 11330032) containing 0.1% casein (Sigma Cat# C3400), subjected to the adjustment of cell density with the assay buffer, and seeded in a 384-well plate (Sigma Cat# CLS4514) at a density of 5000 cells/5 µL/well. Then 2.5 µL of an IBMX working solution (Sigma Cat# I7018) prepared in a buffer (the final concentration of IBMX was 0.5 mM) and 2.5 µL of polypeptide samples diluted in a gradient were added to each well, and the plate was centrifuged at 1000 rpm for 1 min, shaken for 30 s for mixing well, and left to stand for incubation at room temperature for 30 min. Detection was performed using the Cisbio cAMP-Gs Dynamic kit (Cisbio Cat# 62 AM4PEC), and cAMP-d2 and Anti-cAMP-Eu3+-Cryptate were each subjected to a 20-fold dilution using cAMP Lysis & Detection Buffer and each mixed well. 5 µL of diluted cAMP-d2 solution was added to each well, followed by the addition of 5 µL of diluted Anti-cAMP- Eu3+-Cryptate solution, and the mixture was shaken for 30 s for mixing well and then incubated at room temperature for 1 h in the dark.

### 3. Data processing:

HTRF signal reading was performed using a Biotek Synergy H1 microplate reader at an excitation wavelength of 320 nm and emission wavelengths of 620 nm and 665 nm. The signal ratios (665 nm/620 nm*10,000) were calculated and fitted non-linearly to sample concentrations in GraphPad Prism 6 using a four-parameter equation to obtain EC₅₀ values, with the specific data shown in Tables 5 and 6 below.

**Table 5. Determination results of agonist activity against human GLP-1R and human GIPR**

| Compound | Activity against human GLP-1R (EC₅₀ nM) | Activity against human GIPR (EC₅₀ nM) |
|---|---|---|
| Natural GLP-1 | 0.010 | N/A |
| Natural GIP | N/A | 0.011 |
| Semaglutide | 0.024 | >10 |
| LY3298176 | 0.13 | 0.056 |
| 7# | 0.021 | 0.11 |

**Table 6. Determination results of agonist activity against human GLP-1R and human GIPR**

| Compound | Activity against human GLP-1R (EC₅₀ nM) | Activity against human GIPR (EC₅₀ nM) |
|---|---|---|
| Natural GLP-1 | 0.006 | N/A |
| Natural GIP | N/A | 0.006 |
| Semaglutide | 0.014 | >10.0 |
| LY3298176 | 0.078 | 0.031 |
| 9# | 0.049 | 0.040 |
| 10# | 0.065 | 0.056 |
| 12# | 0.030 | 0.170 |
| 13# | 0.017 | 0.130 |
| 14# | 0.013 | 0.130 |
| 15# | 0.015 | 0.230 |
| 16# | 0.029 | 0.095 |
| 17# | 0.022 | 0.110 |
| 18# | 0.013 | 0.060 |

### 4. Experimental conclusion:

Through the design of the polypeptide skeleton and the subsequent site-directed fatty acid modification, the compounds of the present disclosure have stronger agonist activity against GLP-1/GIPR than many GLP-1/GIPR dual-agonist polypeptides in the art and thus have greater potential for treating metabolic diseases. In addition, LY3298176 shows preferential activity against GIPR, while the compounds 12#-18# of the present disclosure show preferential activity against GLP-1R.

### Example 5. Stability Test of Some of Compounds of the Present Disclosure

Stability in plasma is important for therapeutic polypeptide drugs, since the polypeptide drugs are likely to be sensitive to polypeptide hydrolases and protein hydrolases in plasma. The half-life and efficacy of polypeptides that are unstable in plasma will be affected.

### 1. Experimental objective:

This experiment was intended to test the stability of some of the compounds of the present disclosure in human plasma.

### 2. Experimental procedures:

5 µL of each of samples at concentrations of 20 ng/mL, 50 ng/mL, 100 ng/mL, 200 ng/mL, 500 ng/mL, 1000 ng/mL, 2000 ng/mL, 5000 ng/mL, and 10000 ng/mL was added to 45 µL of human plasma. The content of the compounds in the samples was determined by the LC-MS method and a standard curve was formed. 5 µL of a 1 mg/mL polypeptide solution was added to 45 µL of human plasma. Five samples were prepared for each test compound, and the samples were taken at 0 min, 30 min, 60 min, 120 min, and 240 min, respectively, and determined for the content of the retained compound by the LC-MS method. With the content at 0 min as the standard (100%), the relative content of the retained compounds in the samples at other time points was calculated. The LC-MS method for detecting the compounds was as follows: a 5% acetonitrile solution was prepared as solution A, a 95% acetonitrile solution was prepared as solution B, solution gradients were formed at a flow rate of 0.6 mL/min according to the time points and solution proportions shown in Table 9, and 15 µL of the sample was injected, and the content of the compounds was determined using a Raptor Biphenyl 2.7 µm detection column; see Table 7.

**Table 7. Test time points and solution proportions**

| Time (min) | A (%) | B (%) |
|---|---|---|
| 0.20 | 95.0 | 5.00 |
| 1.70 | 5.00 | 95.0 |
| 2.00 | 5.00 | 95.0 |
| 2.01 | 95.0 | 5.00 |
| 2.50 | 95.0 | 5.00 |

### 3. Experimental results:

The data for the stability of some of the compounds of the present disclosure in plasma are shown in Table 8 below.

**Table 8. Experimental results of the stability of the compounds in plasma**

| Compound | Relative content of compounds retained in plasma (%) | | | | |
|---|---|---|---|---|---|
| | 0 min | 30 min | 60 min | 120 min | 240 min |
| LY3298176 | 100.00 | 102.89 | 114.76 | 117.59 | 113.35 |
| 7# | 100.00 | 101.45 | 101.66 | 103.28 | 102.15 |

### Conclusion:

It was found by study that compound **7#** of the present disclosure has similar stability (relative content > 90%) in human plasma compared with compound LY3298176 at the 4-h time point.

### Example 6. Pharmacokinetic Properties of Some of Compounds of the Present Disclosure in Mice

Plasma stability is one of the factors that affect the pharmacokinetics of polypeptide drugs. The pharmacokinetics of polypeptide drugs *in vivo* is also affected by factors such as absorption and clearance of the polypeptide drugs *in vivo.*

### 1. Experimental objective:

This experiment was intended to study the pharmacokinetic behavior of the compounds of the present disclosure in Balb/c mice (plasma) after a single intravenous injection by taking the mice as test animals.

### 2. Experimental procedures:

Male Balb/c mice weighing 18-30 g and aged 7-9 weeks were purchased from Shanghai Jiesijie Laboratory Animal Co., Ltd. After compound **7#** was prepared in a buffer containing 20 mM citric acid (pH = 7.0), compound **7#** was intravenous injected into mice at a dose of 30 nmol/kg body weight via tail vein, and 0.2 mL of blood was separately collected at time points of 0 h, 0.083 h, 0.25 h, 0.5 h, 1 h, 2 h, 4 h, 6 h, 8 h, 24 h, and 32 h. The collected blood of mice was centrifuged at 6000 rpm for 6 min at 4 °C to separate the plasma. The content of compound **7#** in plasma of mice was assayed by the experimental procedures of Example 3.3.

### 3. Experimental results:

Through the above experimental procedures, the specific data are shown in Table 9 below.

**Table 9. Pharmacokinetic behavior after a single intravenous injection in mice (plasma)**

| PK parameters | Unit | Compound 7# |
|---|---|---|
| T_{1/2} | h | 13.0 |
| AUCinf | h*ng/mL | 16133 |

### 4. Experimental conclusion:

It was found by study that compound **7#** of the present disclosure has good pharmacokinetic properties after intravenous injection into mice, indicating that this compound is advantageous in treating diseases, for example, being able to support subcutaneous injection once a week in humans.

### Example 7. Pharmacokinetic Properties of Some of Compounds of the Present Disclosure in Mice

### 1. Experimental objective:

This experiment was intended to study the pharmacokinetic behavior of the compounds of the present disclosure in Balb/c mice (plasma) after a single subcutaneous injection by taking the mice as test animals.

### 2. Experimental procedures:

Male Balb/c mice weighing 18-30 g and aged 7-9 weeks were purchased from Shanghai Jiesijie Laboratory Animal Co., Ltd. After compound **7#** was prepared in a buffer containing 20 mM citric acid (pH = 7.0), compound **7#** was subcutaneously injected into mice at a dose of 30 nmol/kg body weight via left side of abdomen, and 0.2 mL of blood was separately collected at time points of 0 h, 0.083 h, 0.25 h, 0.5 h, 1 h, 2 h, 4 h, 6 h, 8 h, 24 h, and 32 h. The collected blood of mice was centrifuged at 6000 rpm for 6 min at 4 °C to separate the plasma. The content of compound 7# in plasma of mice was assayed by the experimental procedures of Example 5.2.

### 3. Experimental results:

Through the above experimental procedures, the specific data are shown in Table 10 below.

**Table 10. Pharmacokinetic results for compound 7# in mice**

| PK parameters | Unit | Compound 7# |
|---|---|---|
| T_{1/2} | h | 10.1 |
| AUCinf | h*ng/mL | 14488 |

### 4. Experimental conclusion:

It was found by study that the compound of the present disclosure has good pharmacokinetic properties after subcutaneous injection into mice, indicating that this compound is advantageous in treating diseases, for example, being able to support subcutaneous injection once a week in humans.

### Example 8. In Vivo Efficacy of Some of Compounds of the Present Disclosure

### 1. Experimental objective:

This experiment was intended to test the regulatory effect of some of the compounds of the present disclosure and compound LY3298176 on blood glucose in normal mice after a single subcutaneous administration.

### 2. Experimental procedures:

Male C57BL/6 mice aged 10-12 weeks were purchased from Shanghai Jiesijie Laboratory Animal Co., Ltd. The C57BL/6 mice were subcutaneously injected with compound **7#** or compound LY3298176 (dose: 10 nmol/kg body weight) and a control buffer, and then fasted without water deprivation. 18 h later, a glucose solution at a concentration of 0.2 g/mL was intraperitoneally injected. Blood glucose values were measured by collecting blood from the tail of mice at time points of 0 min, 15 min, 30 min, 60 min, and 120 min according to the experimental design. The specific procedures were as follows: the mouse was physically immobilized with the tail exposed, a little part was cut off at the tail end, then the tail was squeezed to bleed, and blood glucose was determined using a Roche active glucometer after the 1st drop of blood was discarded. The area under the blood glucose curve (AUC) was calculated from the results of all time points.

### 3. Experimental results:

Through the above experimental procedures, the specific data are shown in Table 11 below.

**Table 11. Change in blood glucose values of mice after a single subcutaneous administration**

| Test compounds | Dose | Blood glucose (mMol/L, mean±SD) | | | | | AUC (mMol/L.hr) |
|---|---|---|---|---|---|---|---|
| | | 0 min | 15 min | 30 min | 60 min | 120 min | |
| Placebo | - | 5.3±0.6 | 20.5±2.0 | 24.0±1.4 | 19±1.3 | 10.9±1.2 | 34.5±2.4 |
| 7# | 10nmol/kg | 4.4±0.8 | 6.7±0.8 | 6.2±1.3 | 5.7±1.2 | 3.8±1.1 | 10.7±1.8 |
| LY3298176 | 10nmol/kg | 3.2±0.2 | 9.1±1.3 | 8±1.4 | 6.4±1.0 | 4.5±0.7 | 12.7±1.6 |

### 4. Experimental conclusion:

In this experiment, compound **7#** of the present disclosure shows significant blood glucose-lowering effect on normal mice at a dose of 10 nmol/kg body weight, with the area under the blood glucose curve of compound **7#** group reduced by more than 60% compared with that of placebo (i.e., blank vehicle).

### Example 9. Body Weight-Reducing Efficacy of Some of Compounds of the Present Disclosure

### 1. Experimental objective:

This experiment was intended to test the regulatory effect of the numbered compounds on the body weight of diet-induced-obesity mice after subcutaneous administration.

### 2. Experimental procedures:

High-fat food-induced-obesity male C57BL/6 mice (weighing 35-55 g, aged 10-12 weeks, purchased from Shanghai Jiesijie Laboratory Animal Co., Ltd.) were tested. The diet-induced-obesity C57BL/6 mice were subcutaneously injected with compound LY3298176 (10 nmol/kg body weight), compound 7# (10 nmol/kg body weight), and compound 18# (three doses of 3 nmol/kg, 10 nmol/kg, and 100 nmol/kg body weight, administered once every 3 days). According to the experimental design, the body weight of each mouse was measured and recorded on day 0, day 3, day 6, and so on to day 27, the average body weight of each group of mice was calculated, and body weight change curves were plotted by taking the body weight on the first day as the standard. At the end, the fat and other visceral organs of each part of the mice were taken out and weighed, and the viscera/brain ratio for fat in each part of each mouse was calculated. The effect of the drug on the fat was determined by comparing the change in the viscera/brain ratio for fat of different parts of each group of mice.

### 3. Experimental results:

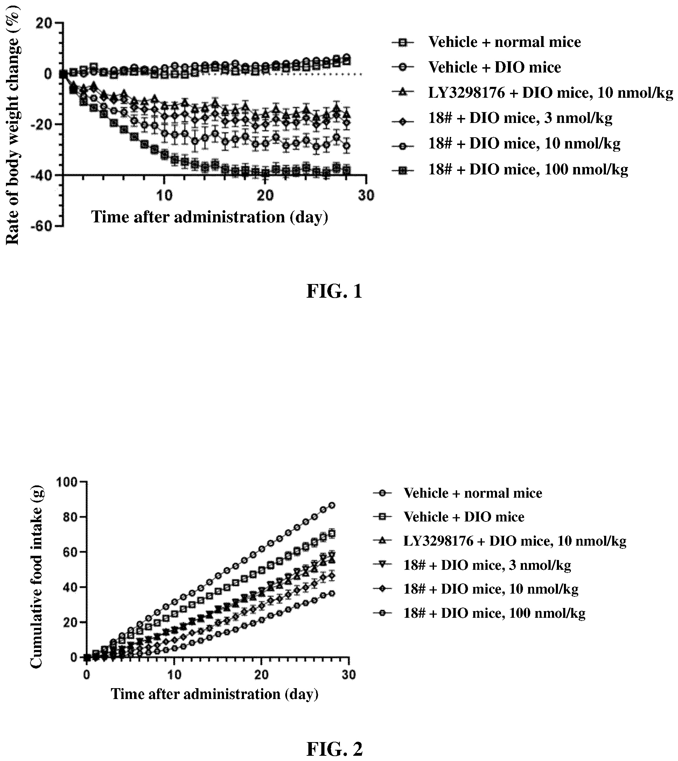

Through the above experimental procedures, the specific data are shown in Tables 12 to 14 below and FIG. 1.

**Table 12. Body weight-reducing effect of compounds on induced-obesity mice**

| | Days | Test compound (dose) | |
|---|---|---|---|
| | | Placebo (-) | 7# (10 nmol/kg) |
| Body weight change (%, mean±SD) | Day 1 | 0 | 0 |
| | Day 4 | -0.9±1.6 | -11.0±1.0 |
| | Day 7 | -2.6±1.5 | -17.6±2.6 |
| | Day 10 | -3.0±2.4 | -22.6±5.9 |
| | Day 13 | -3.5±3.7 | -22.3±6.3 |
| | Day 16 | -2.7±4.8 | -22.7±6.9 |
| | Day 19 | -2.8±6.7 | -25.9±6.2 |
| | Day 22 | -2.5±8.4 | -23.9±5.6 |
| | Day 25 | -1.4±8.9 | -23.9±5.6 |
| | Day 28 | -2.1±9.6 | -25.5±5.3 |

### 4. Experimental conclusion:

In this experiment, at the doses of 3 nmol/kg, 10 nmol/kg, and 100 nmol/kg, the compounds 7# and 18# of the present disclosure show significant body weight-reducing effect on high-fat food-induced-obesity mice and exhibit significant dose dependence. The body weight of mice in 10 nmol/kg dose test group of the compound 18# was reduced by more than 20.0% on day 27, while the body weight of mice in the same dose test group of the control compound LY3298176 was reduced by about 13.4%. In addition, the content of fat of each part (except scapular fat) of mice in all dose test groups of the compound 18# was significantly reduced relative to that of the placebo (i.e., blank vehicle) group.

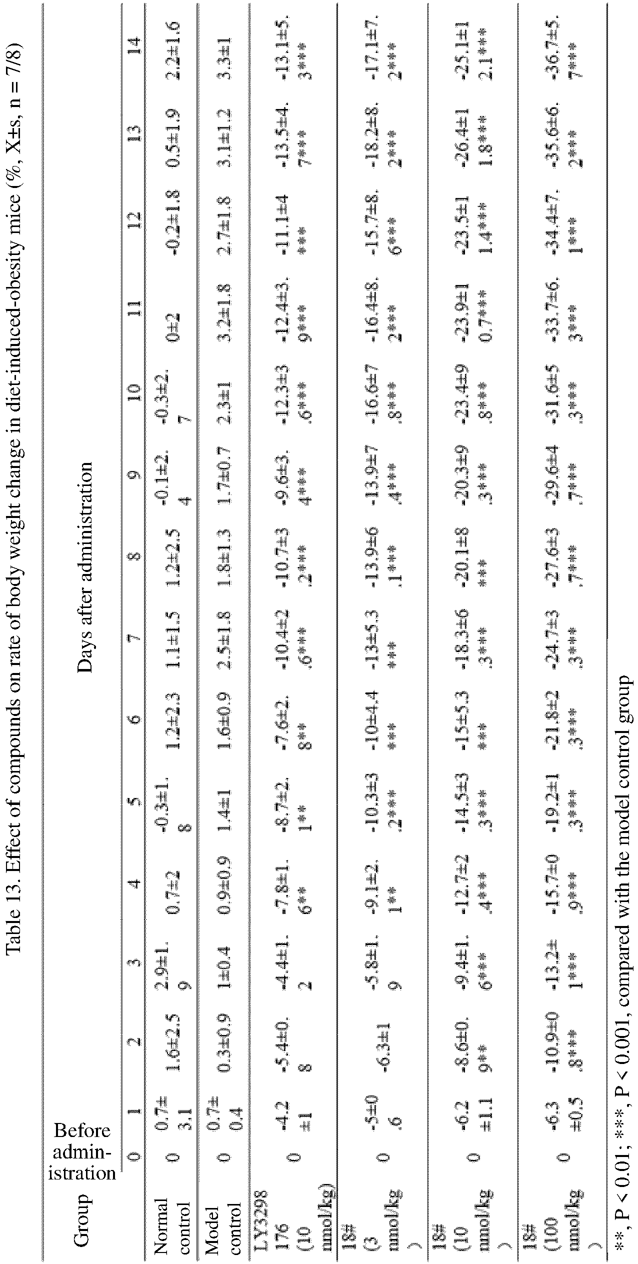

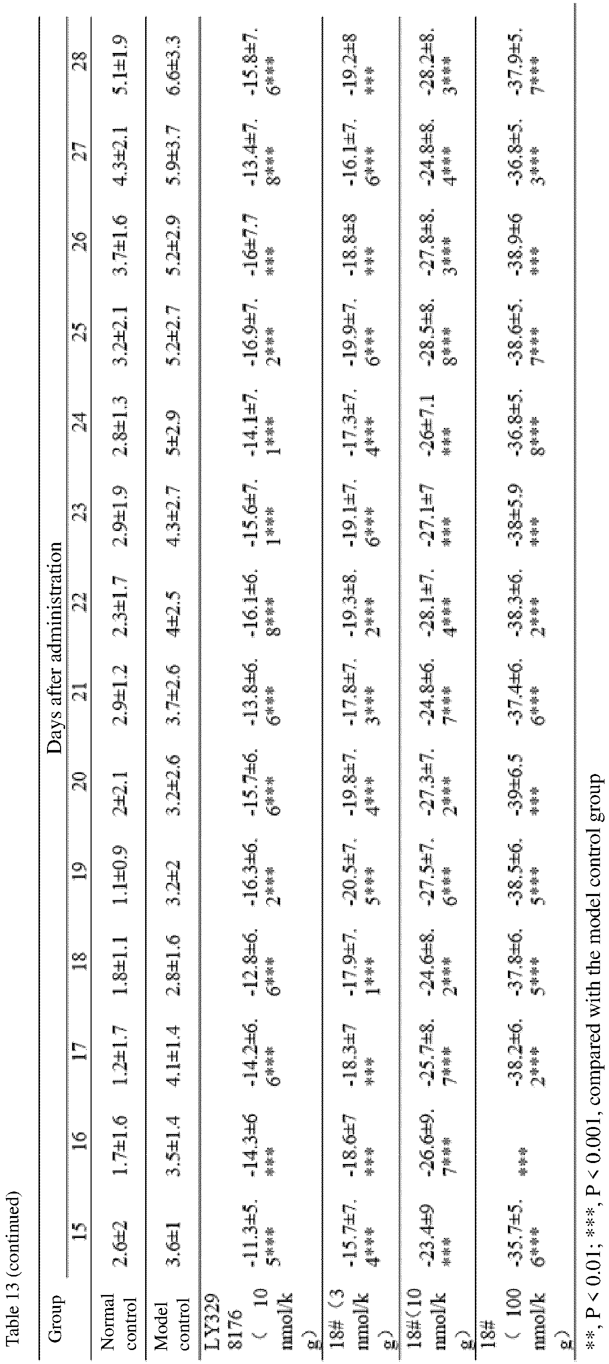

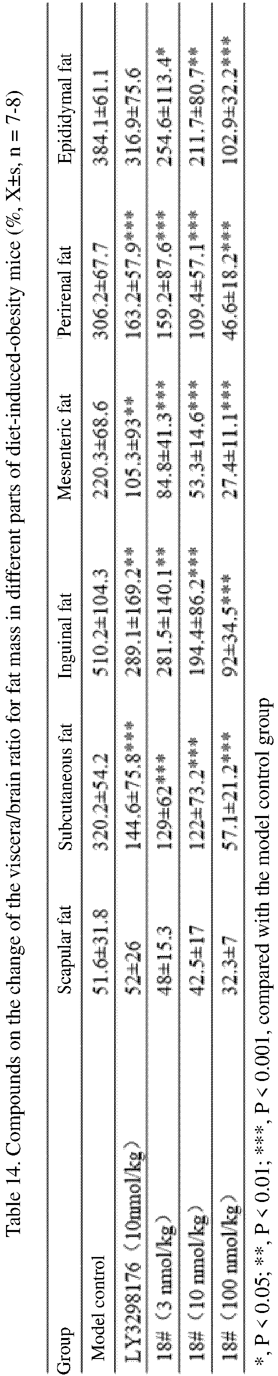

### Example 10. Effect of Compounds of the Present Disclosure on Food Intake of Mice

The food intake of mice in each group was measured daily during the experiment. The results are shown in Table 1 and FIG. 2.

The average daily food intake of DIO (diet-induced obesity) mice in the model control group was 2.5 g throughout the experiment. After subcutaneous injection of the compound 18# or compound LY3298176 at different doses, the food intake of mice in all groups was reduced to different extents.

On the first day after the administration, the food intake of mice in each administration group was significantly reduced, with the food intake of mice in 3 nmol/kg, 10 nmol/kg, and 100 nmol/kg dose groups of the compound 18# being 0.6 g, 0.3 g, and 0.2 g, respectively, which was significantly different from that of the model control group (2.5 g) and showed a better dose-effect relationship.

The cumulative food intake of the mice in the model control group within 5 days after the administration was 12.8 g, while the cumulative food intake of the mice in the 3 nmol/kg, 10 nmol/kg, and 100 nmol/kg dose groups of the compound 18# within 5 days after the administration was 7.2 g, 3.9 g, and 1.8 g, respectively, which was significantly lower than that of the model control group and showed a better dose-effect relationship. Daily food intake of mice in each administration group began to decrease on day 1 and began to restore on days 2 and 3 after each administration. Daily food intake showed an overall upward recovery trend during the administration. 28 days after the administration, the cumulative food intake of three dose groups of the compound 18# was 58.2 g, 46.8 g, and 36.7 g, respectively, which was significantly lower than that of the model control group (70.8 g) and showed a better dose dependence. Therefore, the compound 18# can significantly reduce food intake of DIO mice.

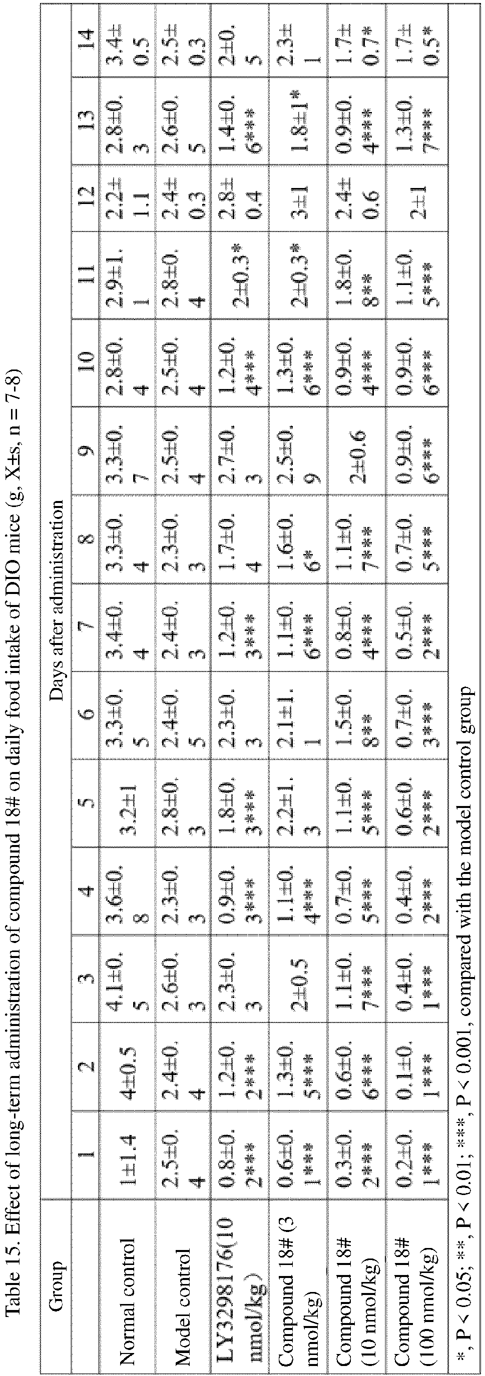

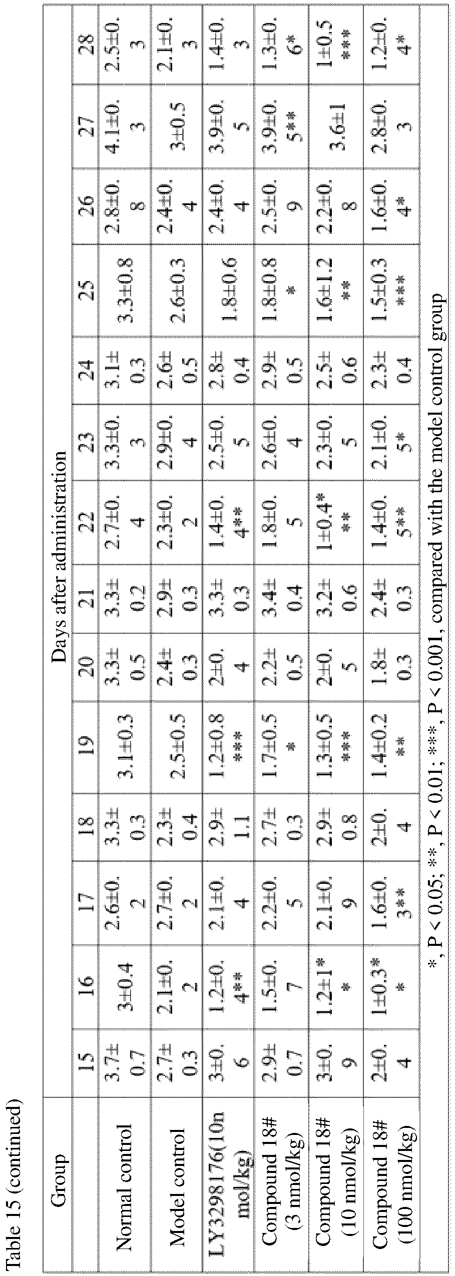

### Example 11. Improvement Effect of Some of Compounds of the Present Disclosure on Glucose Metabolism Level of db/db Mice

### 1. Experimental objective:

This experiment was intended to test the improvement effect of the numbered compound on the glucose metabolism level of db/db mice after subcutaneous administration.

### 2. Experimental procedures:

C57BL/KsJ-db/db mice were subcutaneously injected with blank vehicle (20 mM sodium citrate + 0.05% Tween-80, pH 7.5), compound LY3298176 (100 nmol/kg body weight), and compound 18# (three doses of 10 nmol/kg body weight, 30 nmol/kg body weight, and 100 nmol/kg body weight) on days 0, 3, 7, 10, 14, 17, 21, 24, and 27. Each administration group had 10 db/db mice. According to the experimental design, tail vein blood was collected by needle pricking on days 0, 7, 14, 21, and 28 and determined for fasting blood glucose levels with a glucometer and glucose dipsticks, and the mice were fasted 6 h prior to the blood collection at each time point. Tail vein blood was collected by needle pricking on days 3, 10, 17, 24, and 27 and randomly determined for blood glucose levels with a glucometer. Finally, at the end of the experiment on day 28, all the animals in the administration groups were subjected to 2-5% isoflurane inhalation anesthesia, and 100 µL of whole blood was collected through the orbit of each mouse using an EDTA-K2 anticoagulation tube and used for the determination of glycated hemoglobin.

### 3. Experimental results:

Through the above experimental procedures, the specific data are shown in Tables 16 to 18 below.

**Table 16. Effect of long-term administration of compound 18# on fasting blood glucose of db/db mice**

| Administration group | Concentration of fasting blood glucose (mMol/L, mean±SD) | | | | |
|---|---|---|---|---|---|
| | Day 0 | Day 7 | Day 14 | Day 21 | Day 28 |
| Blank control | 14.25±1.27 | 17.92±1.33 | 22.89±1.88 | 24.95±1.52 | 25.94±1.32 |
| LY3298176 (100 nmol/kg) | 14.35±1.41 | 7.44±0.76 | 7.56±0.88 | 9.42±1.67 | 9.89±1.28*** |
| #18 (10 nmol/kg) | 14.77±1.30 | 6.05±0.42 | 6.30±0.46 | 7.89±0.81 | 9.41±0.97*** |
| #18 (30 nmol/kg) | 14.13±1.32 | 6.21 ±0.26 | 6.40±0.57 | 7.03±0.52 | 9.68±1.03*** |
| #18 (100 nmol/kg) | 14.67±1.46 | 5.85±0.33 | 6.25±0.32 | 6.13±0.19 | 7.89±0.41*** |

| | | | | | |
|---|---|---|---|---|---|
| ***: p < 0.001 vs. blank control group. | | | | | |

**Table 17. Effect of long-term administration of compound 18# on random blood glucose of db/db mice**

| Administration group | Concentration of random blood glucose (mMol/L, mean±SD) | | | |
|---|---|---|---|---|
| | Day 0 | Day 10 | Day 17 | Day 24 |
| Blank control | 23.06±0.97 | 26.40±0.90 | 27.64±1.15 | 30.22±0.74 |
| LY3298176 (100 nmol/kg) | 18.60±1.52 | 17.10±1.96 | 17.98±1.37 | 20.70±1.27*** |
| #18 (10 nmol/kg) | 20.42±1.56 | 20.66±1.48 | 18.91±1.33 | 21.17±2.07** |
| #18 (30 nmol/kg) | 16.73±1.59 | 15.88±1.86 | 17.30±1.17 | 17.43±1.92*** |
| #18 (100 nmol/kg) | 9.11±1.25 | 12.34±1.12 | 11.89±1.15 | 11.51±0.95*** |

| | | | | |
|---|---|---|---|---|
| **: p < 0.01 vs. blank control group; ***: p < 0.001 vs. blank control group. | | | | |

**Table 18. Effect of long-term administration of compound 18# on glycated hemoglobin level of db/db mice**

| Administration group | Glycated hemoglobin (%, mean±SD) |
|---|---|
| Blank control | 6.54±0.17 |
| LY3298176 (100 nmol/kg) | 4.58±0.23** |
| #18 (10 nmol/kg) | 4.71±0.23*** |
| #18 (30 nmol/kg) | 4.53±0.17*** |
| #18 (100 nmol/kg) | 3.78±0.13*** |

| | |
|---|---|
| **: p < 0.01 vs. blank control group; ***: p < 0.001 vs. blank control group. | |

### 4. Experimental conclusion:

In this experiment, at the doses of 10 nmol/kg, 30 nmol/kg, and 100 nmol/kg, the compound 18# of the present disclosure shows excellent improvement effect on the glucose metabolism level of db/db mice and shows significant dose dependence. The glycated hemoglobin level of the 100 nmol/kg dose group of the compound 18# was 3.78% at the end of the experiment, while the glycated hemoglobin level of the same dose group of the control compound LY3298176 was 4.58%. Therefore, the efficacy of the compound 18# in improving the glucose metabolism level of db/db mice is significantly better than that of the control compound LY3298176 at the same dose.

### GLP-1 analogs used in Examples 12-16 are all compound 18#.

### Example 12. Preparation Process for Pharmaceutical Composition

The pharmaceutical composition of the present disclosure can be prepared according to the following process:
Step 1: preparation
   1. Preparation of solution 1: an appropriate amount of water for injection was added to a container and disodium hydrogen phosphate at an amount specified in the formula was dissolved, and the GLP-1 analog at an amount specified in the formula was added or added last.
   2. Preparation of solution 2: propylene glycol and phenol each at an amount specified in the formula were weighed out separately and added to water for injection in another container, and the mixture was stirred until dissolution.
   3. The solution 2 was added to the solution 1, and the mixture was stirred for mixing well. The pH of the drug liquid was adjusted to 7.4-7.8 by using a hydrochloric acid solution or a sodium hydroxide solution, water for injection was added additionally to reach the amount specified in the formula, and the resulting mixture was stirred.
Step 2: sterilization and filtration

After the preparation, the drug liquid was filtered through a sterilizing filter element (pore size: 0.22 µm).

The sterilized and filtered drug liquid obtained in step 2 was vialed at 1.57-1.67 mL/vial (target filling amount being 1.62 mL), followed by capping and visually inspecting.

### Example 13. Screening for pH of Formulation

The formulation shown in Table 19 was prepared, and the stability of the formulation was examined at pH 6.5, 7.0, 7.5, 8.0, 8.5, and 9.0 using the appearance, related substance, and oligopeptide as the indexes, and the results are shown in Table 20.

**Table 19. Exemplary formulation in unit dose**

| **Component** | **Amount** | **Ratio (w/v%)** |
|---|---|---|
| GLP-1 analog | 5.00 mg | 0.500 |
| Disodium hydrogen phosphate | 0.71 mg | 0.071 |
| Propylene glycol (injection grade) | 14.0 mg | 1.400 |
| Phenol | 5.50 mg | 0.550 |
| Hydrochloric acid | q.s. | q.s. |
| Sodium hydroxide | q.s. | q.s. |
| Water for injection | Adding to bring the volume to 1 mL | N/A |

**Table 20. Screening results for solution pH**

| **pH** | **Time point** | **Appearance** | **Alkalinity** | **Related substance** | | **Oligopeptide (%)** |
|---|---|---|---|---|---|---|
| | | | | **Maximum single impurity (%)** | **Total impurity (%)** | |
| pH 6.5 | 0 days | Colorless clear liquid | 6.6 | 0.52 | 1.6 | 0.06 |
| | 25 °C-6 days | White turbid liquid | 6.7 | 0.69 | 1.9 | 0.07 |
| | 25 °C-10 days | White turbid liquid | 6.8 | 0.73 | 2.0 | 0.08 |
| | 40 °C-6 days | White turbid liquid | 6.8 | 0.89 | 2.6 | 0.09 |
| | 40 °C-10 days | White turbid liquid | 6.8 | 1.04 | 2.7 | 0.15 |
| pH 7.0 | 0 days | Colorless clear liquid | 7.0 | 0.52 | 1.6 | 0.06 |
| | 25 °C-6 days | Colorless clear liquid | 7.0 | 0.70 | 1.9 | 0.07 |
| | 25 °C-10 days | Colorless clear liquid | 7.0 | 0.74 | 2.0 | 0.07 |
| | 40 °C-6 days | Colorless clear liquid | 7.0 | 0.95 | 2.3 | 0.09 |
| | 40 °C-10 days | Colorless clear liquid | 7.0 | 1.23 | 2.6 | 0.14 |
| pH 7.5 | 0 days | Colorless clear liquid | 7.5 | 0.53 | 1.6 | 0.06 |
| | 25 °C-6 days | Colorless clear liquid | 7.5 | 0.71 | 2.0 | 0.08 |
| | 25 °C-10 days | Colorless clear liquid | 7.5 | 0.75 | 2.0 | 0.09 |
| | 40 °C-6 days | Colorless clear liquid | 7.5 | 1.0 | 2.7 | 0.15 |
| | 40 °C-10 days | Colorless clear liquid | 7.5 | 1.3 | 3.1 | 0.27 |
| pH 8.0 | 0 days | Colorless clear liquid | 8.0 | 0.55 | 1.8 | 0.07 |
| | 25 °C-6 days | Colorless clear liquid | 8.0 | 0.67 | 1.9 | 0.13 |
| | 25 °C-10 days | Colorless clear liquid | 8.0 | 0.75 | 2.1 | 0.16 |
| | 40 °C-6 days | Colorless clear liquid | 8.0 | 0.94 | 3.0 | 0.30 |
| | 40 °C-10 days | Colorless clear liquid | 8.0 | 1.2 | 5.0 | 0.60 |
| pH 8.5 | 0 days | Colorless clear liquid | 8.5 | 0.52 | 1.6 | 0.07 |
| | 25 °C-6 days | Colorless clear liquid | 8.5 | 0.69 | 2.2 | 0.24 |
| | 25 °C-10 days | Colorless clear liquid | 8.5 | 0.73 | 2.6 | 0.39 |
| | 40 °C-6 days | Colorless clear liquid | 8.5 | 0.83 | 3.6 | 0.73 |

| **pH** | **Time point** | **Appearance** | **Alkalinity** | **Related substance** | | **Oligopeptide** |
|---|---|---|---|---|---|---|
| | 40 °C-10 days | Colorless clear liquid | 8.5 | 1.2 | 8.2 | 1.4 |
| **pH** 9.0 | 0 days | Colorless clear liquid | 9.0 | 0.52 | 1.6 | 0.07 |
| | 25 °C-6 days | Colorless clear liquid | 9.0 | 0.64 | 2.8 | 0.59 |
| | 25 °C-10 days | Colorless clear liquid | 9.0 | 0.76 | 3.9 | 0.99 |
| | 40 °C-6 days | Colorless clear liquid | 9.0 | 2.28 | 8.9 | 1.8 |
| | 40 °C-10 days | Colorless clear liquid | 9.0 | 5.06 | 17.3 | 3.2 |

The results of the stability test show that for the intermediate solution, the formulation at pH 6.5 was turbid, the formulations at pH 8.5 or more showed relatively great increase in related substance, and the formulations at pH 7.0, pH 7.5, and pH 8.0 showed relatively good stability. Based on comprehensive consideration, the acceptable range of the pH value of the intermediate solution is determined to be 7.0-8.0.

### Example 14. Screening for Concentration of Buffer

The stability of the formulations was examined in the case that the concentrations of disodium hydrogen phosphate were 0 mM, 5 mM, 10 mM, and 40 mM, using the appearance, related substance, and oligopeptide as the indexes, and the results are shown in Table 21.

**Table 21. Screening results for concentration of buffer**

| **Disodium hydrogen phosphate Concentration** | **Time point** | **Appearance** | **Alkalinity** | **Related substance** | | **Oligopeptide (%)** |
|---|---|---|---|---|---|---|
| | | | | **Maximum single impurity (%)** | **Total impurity (%)** | |
| 0 mM | 0 days | Colorless clear liquid | 7.6 | 0.52 | 1.5 | 0.07 |
| | 25 °C-6 days | Colorless clear liquid | 7.6 | 0.71 | 2.0 | 0.09 |
| | 25 °C-10 days | Colorless clear liquid | 7.5 | 0.74 | 2.0 | 0.09 |
| | 40 °C-6 days | Colorless clear liquid | 7.5 | 1.0 | 2.6 | 0.12 |
| | 40 °C-10 days | Colorless clear liquid | 7.5 | 1.3 | 3.2 | 0.19 |
| 5 mM | 0 days | Colorless clear liquid | 7.5 | 0.53 | 1.6 | 0.06 |
| | 25 °C-6 days | Colorless clear liquid | 7.5 | 0.71 | 2.0 | 0.08 |
| | 25 °C-10 days | Colorless clear liquid | 7.5 | 0.75 | 2.0 | 0.09 |
| | 40 °C-6 days | Colorless clear liquid | 7.5 | 1.0 | 2.7 | 0.15 |
| | 40 °C-10 days | Colorless clear liquid | 7.5 | 1.3 | 3.1 | 0.27 |
| 10 mM | 0 days | Colorless clear liquid | 7.6 | 0.53 | 1.6 | 0.07 |
| | 25 °C-6 days | Colorless clear liquid | 7.6 | 0.73 | 2.2 | 0.09 |
| | 25 °C-10 days | Colorless clear liquid | 7.6 | 0.75 | 2.0 | 0.11 |
| | 40 °C-6 days | Colorless clear liquid | 7.6 | 1.0 | 2.8 | 0.18 |
| | 40 °C-10 days | Colorless clear liquid | 7.5 | 1.3 | 3.4 | 0.27 |
| 40 mM | 0 days | Colorless clear liquid | 7.6 | 0.55 | 1.8 | 0.07 |
| | 25 °C-6 days | Colorless clear liquid | 7.6 | 0.71 | 2.0 | 0.09 |
| | 25 °C-10 days | Colorless clear liquid | 7.6 | 0.75 | 2.1 | 0.13 |
| | 40 °C-6 days | Colorless clear liquid | 7.6 | 0.99 | 2.9 | 0.23 |
| | 40 °C-10 days | Colorless clear liquid | 7.6 | 1.3 | 3.8 | 0.35 |

The experimental results show that the oligopeptide increased slightly faster in the formulation containing disodium hydrogen phosphate than in the formulation without disodium hydrogen phosphate (0 mM), wherein the 40 mM formulation containing disodium hydrogen phosphate at a relatively higher concentration showed a relatively great increase in oligopeptide, the 5 mM and 10 mM formulations showed a relatively small increase in oligopeptide, and the oligopeptide in each formulation was within the limits.

### Example 15. Screening for Amount of Bacteriostatic Agent

A GLP-1 analog injection can be used in a single dose form or a multi-dose packaging form, and in order to ensure the requirement on sterility in use, a bacteriostatic agent needs to be added, and the bacteriostatic agent is used in an amount capable of inhibiting the growth of microorganisms in the injection. Phenol at a concentration of 5.5 mg/mL was selected; by taking this concentration as 100%, formulations at relative concentrations of 80%, 100%, and 120% were prepared for examination of bacteriostatic effect. The results are shown in Table 22.

**Table 22. Screening results for amount of bacteriostatic agent**

| Phenol content: 4.4 mg/mL | | Reduced 1g value¹ | | | | |
|---|---|---|---|---|---|---|
| | | 6h | 24h | 7d | 14d | 28d |
| Bacteria | *Pseudomonas aeruginosa* | 1.25 | 3.66 | 5.66 | - | 5.66 |
| | *Staphylococcus aureus* | 1.22 | 2.98 | 5.76 | - | 5.76 |
| Fungi | *Candida albicans* | - | - | 3.58 | 5.11 | 5.11 |
| | *Aspergillus niger* | - | - | 5.08 | 5.08 | 5.08 |

| Phenol content: 5.5 mg/mL | | Reduced 1g value | | | | |
|---|---|---|---|---|---|---|
| | | 6h | 24h | 7d | 14d | 28d |
| Bacteria | *Pseudomonas aeruginosa* | 2.1 | 5.66 | 5.66 | - | 5.66 |
| | *Staphylococcus aureus* | 2.14 | 4.04 | 5.76 | - | 5.76 |
| Fungi | *Candida albicans* | - | - | 5.11 | 5.11 | 5.11 |
| | *Aspergillus niger* | - | - | 5.08 | 5.08 | 5.08 |

| Phenol content: 6.6 mg/mL | | Reduced 1g value | | | | |
|---|---|---|---|---|---|---|
| | | 6h | 24h | 7d | 14d | 28d |
| Bacteria | *Pseudomonas aeruginosa* | 5.66 | 5.66 | 5.66 | - | 5.66 |
| | *Staphylococcus aureus* | 3.77 | 5.76 | 5.76 | - | 5.76 |
| Fungi | *Candida albicans* | - | - | 5.11 | 5.11 | 5.11 |
| | *Aspergillus niger* | - | - | 5.08 | 5.08 | 5.08 |

| | | | | | | |
|---|---|---|---|---|---|---|
| Note 1: the difference between the lg value of the number of bacteria measured at each interval and the lg value of the number of bacteria inoculated in 1 mL (g) of the test sample. | | | | | | |

The experimental results show that the formulations of GLP-1 analog exhibited bacteriostatic effect when the phenol content was 4.40-6.60 mg/mL, and the antibacterial effect was better when the concentration of phenol was 5.50 mg/mL and 6.60 mg/mL.

### Example 16. Screening for Concentration of GLP-1 Analog

Samples containing GLP-1 analog at concentrations of 2 mg/mL, 4 mg/mL, 5 mg/mL, 6 mg/mL, 8 mg/mL, and 10 mg/mL were prepared separately using propylene glycol as an osmotic pressure regulator, and the formulations are shown in Table 23.

**Table 23. Stability results at 25 °C**

| Concentration of GLP-1 analog | Time point | | Appearance | Alkalinity | Related substance (%) | | Oligopeptide (%) |
|---|---|---|---|---|---|---|---|
| | | | | | Maximum single impurity | Total impurity | |
| 2 mg/mL | Initial | | Colorless clear liquid | 7.6 | 0.65 | 1.8 | 0.07 |
| | 25°C | 1M | Colorless clear liquid | / | 0.86 | 2.1 | 0.20 |
| | | 3M | Colorless clear liquid | 7.5 | 1.4 | 3.8 | 0.48 |
| | | 4M | Colorless clear liquid | 7.5 | 1.7 | 4.4 | 0.65 |
| 4 mg/mL | Initial | | Colorless clear liquid | 7.6 | 0.67 | 2.1 | 0.07 |
| | 25°C | 1M | Colorless clear liquid | 7.5 | 0.87 | 2.1 | 0.14 |
| | | 3M | Colorless clear liquid | 7.5 | 1.4 | 3.9 | 0.27 |
| | | 4M | Colorless clear liquid | 7.5 | 1.7 | 4.1 | 0.39 |
| 5 mg/mL | Initial | | Colorless clear liquid | 7.6 | 0.64 | 2.1 | 0.08 |
| | 25°C | 3M | Colorless clear liquid | 7.6 | 1.4 | 4.1 | 0.21 |
| 6 mg/mL | Initial | | Colorless clear liquid | 7.5 | 0.64 | 1.7 | 0.07 |
| | 25°C | 1M | Colorless clear liquid | 7.5 | 0.78 | 1.8 | 0.12 |
| | | 3M | Colorless clear liquid | 7.5 | 1.4 | 3.7 | 0.23 |
| | | 4M | Colorless clear liquid | 7.5 | 1.7 | 4.2 | 0.28 |
| 8 mg/mL | Initial | | Colorless clear liquid | 7.6 | 0.63 | 0.63 | 1.8 |
| | 25°C | 1M | Colorless clear liquid | 7.6 | 0.86 | 2.2 | 0.12 |
| | | 3M | Colorless clear liquid | 7.6 | 1.4 | 3.7 | 0.22 |
| | | 4M | Colorless clear liquid | 7.6 | 1.7 | 4.3 | 0.28 |
| 10 mg/mL | Initial | | Colorless clear liquid | 7.6 | 0.63 | 0.63 | 1.8 |
| | 25°C | 1M | Colorless clear liquid | 7.6 | 0.85 | 2.1 | 0.13 |
| | | 3M | Colorless clear liquid | 7.6 | 1.4 | 3.7 | 0.22 |
| | | 4M | Colorless clear liquid | 7.6 | 1.7 | 4.3 | 0.28 |

As can be seen from the data in the table above:
(1) the samples at all concentrations were colorless clear liquid at first, which showed that the GLP-1 analog was well dissolved, and the samples at all concentrations showed no significant difference in the pH, related substance, and oligopeptide;
(2) when the samples were stored for 3 months at 25 °C, the samples at all concentrations were all colorless clear liquid and showed no significant difference in the increase trend of the pH value and related substance; the higher the concentration of sample, the lower the oligopeptide content; All the concentration groups showed no significant difference in the increase of impurity, and there was no significant difference at concentrations of 5-10 mg/mL with regard to the increase of impurity and oligopeptide;
(3) in addition, it was found that the samples at all concentrations showed no significant difference in the increase trend of the pH value and related substance after storage at 40 °C for 10 days and 30 days.

Using glycerol and mannitol instead of propylene glycol as the osmotic pressure regulator to prepare GLP-1 analog formulations was tried, and an exemplary formulation in unit dose is shown in Table 24:

**Table 24. Exemplary formulation in unit dose**

| **Concentration of GLP-1 analog** | **6 mg/mL** | | **10 mg/mL** | | | | |
|---|---|---|---|---|---|---|---|
| GLP-1 analog | 6.00 mg | 6.00 mg | 10.0 mg | 10.0 mg | 10.0 mg | 10.0 mg | 10.0 mg |
| Disodium hydrogen phosphate | 0.71 mg | 0.71 mg | 0.71 mg | 0.71 mg | 0.71 mg | 0.71 mg | 0.71 mg |
| Glycerol | 20 mg | / | 20 mg | / | / | / | / |
| Mannitol | / | 45 mg | / | 45 mg | 36.0 mg | 31.5 mg | 27.0 mg |
| Phenol | 5.50 mg | 5.50 mg | 5.50 mg | 5.50 mg | 5.50 mg | 5.50 mg | 5.50 mg |
| Sodium hydroxide | q.s. | q.s. | q.s. | q.s. | q.s. | q.s. | q.s. |
| Hydrochloric acid | q.s. | q.s. | q.s. | q.s. | q.s. | q.s. | q.s. |
| Water for injection (added to bring the volume to) | 1mL | 1mL | 1mL | 1mL | 1mL | 1mL | 1mL |

The stability of the samples at 40 °C was examined and the stability results are shown in Table 25.

**Table 25. Stability results at 40 °C**

| **GLP-1 analog Concentration** | **Osmotic pressure regulator** | **Time point** | | **Appearance** | **Alkalinity** | **Related substance** | | **Oligopeptide %** | **Content%** | **Osmotic pressure (mOsm)** |
|---|---|---|---|---|---|---|---|---|---|---|
| | | | | | | **Maximum single impurity%** | **Total impurity %** | | | |
| **6 mg/mL** | **Glycerol** | 0 days | | Colorless clear liquid | 7.6 | 0.65 | 2.1 | 0.08 | 101.1 | 316 |
| | | 40°C | 10 days | Colorless clear liquid | 7.5 | 1.4 | 4.4 | 0.60 | 96.6 | |
| | | | 30 days | Colorless clear liquid | 7.5 | 2.6 | 10.1 | 1.0 | 91.8 | |
| | **Mannitol** | 0 days | | Colorless clear liquid | 7.7 | 0.63 | 1.9 | 0.08 | 101.1 | 353 |
| | | 40°C | 10 days | Colorless clear liquid | 7.5 | 1.3 | 3.7 | 0.23 | 98.6 | |
| | | | 30 days | Light yellow clear liquid | 7.5 | 2.6 | 8.8 | 0.56 | 93.8 | |
| **10 mg/mL** | **Glycerol** | 0 days | | Colorless clear liquid | 7.7 | 0.63 | 1.9 | 0.08 | 101.1 | 332 |
| | | 40°C | 10 days | Colorless clear liquid | 7.6 | 1.9 | 5.4 | 0.71 | 96.3 | |
| | | | 30 days | Light yellow clear liquid | 7.6 | 2.7 | 11.6 | 1.2 | 91.4 | |
| | **Mannitol** | 0 days | | Colorless clear liquid | 7.7 | 0.63 | 2.0 | 0.08 | 101.9 | 101.8 |
| | | 40°C | 10 days | Colorless clear liquid | 7.6 | 1.3 | 3.9 | 0.24 | 97.4 | |
| | | | 30 days | Light yellow clear liquid | 7.6 | 2.5 | 9.7 | 0.60 | 93.6 | |

As can be seen from the data in the table above:
(1) all the formulations were colorless clear liquid at first, and the GLP-1 analog was well dissolved; all the formulation samples showed no significant difference in the pH, related substance, oligopeptide, and content;
(2) all the formulation samples remained as a clear liquid after storage at 40 °C for 30 days, and no granular or flaky objects were observed, wherein, two formulations using mannitol as the osmotic pressure regulator and one formulation using glycerol as the osmotic pressure regulator were all light yellow, but the color change was not obvious; compared with the formulations using mannitol as the osmotic pressure regulator, the formulations using glycerol as the osmotic pressure regulator showed more remarkable increase in related substance and oligopeptide and more remarkable decrease of content. When the amount of mannitol was 4.5% w/v, the osmotic pressure of the sample was no less than 350 mOsm, which was higher than the range of osmotic pressure suitable for subcutaneous injection (300±30 mOsm). Therefore, the amount of mannitol was reduced to prepare a sample, and an exemplary formulation in unit dose is shown in Table 26. The stability of the sample at 40 °C was examined and the stability results are shown in Table 27.

**Table 26. Exemplary formulation in unit dose**

| **Concentration of GLP-1 analog** | **10 mg/mL** | | |
|---|---|---|---|
| GLP-1 analog | 10.0 mg | 10.0 mg | 10.0 mg |
| Disodium hydrogen phosphate | 0.71 mg | 0.71 mg | 0.71 mg |
| Mannitol | 36.0 mg | 31.5 mg | 27.0 mg |
| Phenol | 5.50 mg | 5.50 mg | 5.50 mg |
| Sodium hydroxide | q.s. | q.s. | q.s. |
| Hydrochloric acid | q.s. | q.s. | q.s. |
| Water for injection (added to bring the volume to) | 1mL | 1mL | 1mL |

**Table 27. Stability results at 40 °C**

| **GLP-1 analog Concentration** | **Osmotic pressure regulator** | **Time point** | | **Appearance** | **Alkalinity** | **Related substance** | | **Oligopeptide%** | **Content%** | **Osmotic pressure (mOsm)** |
|---|---|---|---|---|---|---|---|---|---|---|
| | | | | | | Maximum single impurity% | Total impurity% | | | |
| **10 mg/mL** | **Mannitol (3.60% w/v)** | 0 days | | Colorless clear liquid | 7.5 | 0.64 | 1.8 | 0.07 | 100.4 | 309 |
| | | 40°C | 10 days | Colorless clear liquid | 7.6 | 1.3 | 4.0 | 0.22 | 97.1 | |
| | | | 30 days | Light yellow clear liquid | 7.6 | 2.6 | 7.4 | 0.72 | 89.0 | |
| | **Mannitol (3.15%w/v)** | 0 days | | Colorless clear liquid | 7.7 | 0.66 | 1.9 | 0.07 | 100.8 | 286 |
| | | 40°C | 10 days | Colorless clear liquid | 7.7 | 1.4 | 4.1 | 0.21 | 98.2 | |
| | | | 30 days | Light yellow clear liquid | 7.6 | 2.6 | 7.5 | 0.63 | 92.5 | |
| | **Mannitol (2.70% w/v)** | Can not be completely dissolved | | | | | | | | |

As can be seen from the data in the table above:
(1) in the formulations using mannitol as the osmotic pressure regulator, the GLP-1 analog at a concentration of 10 mg/mL was able to be completely dissolved when the mannitol was either at an amount of 3.15% w/v or 3.6% w/v, and the GLP-1 analog was well dissolved; all the formulation samples showed no significant difference in the pH, related substance, oligopeptide, and content;
(2) the formulation samples containing mannitol at an amount of 3.15% w/v and 3.6% w/v were all light yellow clear liquid after storage at 40 °C for 30 days, but this color change was very insignificant and no visible foreign materials were observed; the formulation containing mannitol at an amount of 3.15% w/v showed slower increase of oligopeptide and decrease of content.

Although the foregoing invention has been described in detail by way of drawings and examples for purposes of clarity of understanding, the description and examples should not be construed as limiting the scope of the present disclosure. The disclosures of all the patents and scientific literature cited herein are clearly incorporated by reference in their entireties.

## Claims

1. A pharmaceutical composition, comprising:
(a) a GLP-1 analog of general formula (I) or a pharmaceutically acceptable salt thereof, 
R1-X1-X2-Glu-Gly-Thr-Phe-Thr-Ser-Asp-X10-Ser-X12-X13-X14-X15-X16-X17-X18-X19-X20
-Glu-Phe-X23-X24-Trp-Leu-X27-X28-X29-X30-Pro-Ser-Ser-Gly-Ala-Pro-Pro-Pro-Ser-R2
(I)
 wherein:
R₁ is H, alkyl, acetyl, formyl, benzoyl, trifluoroacetyl, pGlu, or absent;
R₂ is -NH₂, -OH, or absent;
X₁, X₂, X₁₀, X₁₂, X₁₃, X₁₄, X₁₅, X₁₆, X₁₇, X₁₈, X₁₉, X₂₀, X₂₃, X₂₄, X₂₇, X₂₈, X₂₉, and X₃₀ are independently selected from the group consisting of any natural amino acid residues and any non-natural amino acid residues; and
(b) a buffer, wherein the buffer is selected from any one of an acetate buffer, a histidine salt buffer, a phosphate buffer, a succinate buffer, and a citric acid buffer, preferably a phosphate buffer, and more preferably disodium hydrogen phosphate.

2. The pharmaceutical composition according to claim 1, wherein:
X₁ is Tyr or His; X₂ is Aib or D-Ala; X₁₀ is Val, Tyr, or Y1; X₁₂ is Ser, Ile, or Y1; X₁₃ is Tyr, Ala, or Y1; X₁₄ is Leu, Nle, or Y1; X₁₅ is Asp or Glu; X₁₆ is Arg, Glu, Gly, Lys, Aib, or Y1; X₁₇ is Glu, Ile, Gln, or Y1; X₁₈ is Ala, Aib, or His; X₁₉ is Ala, Aib, or Gin;
X₂₀ is Gin, Glu, or Lys; X₂₃ is Ile or Val; X₂₄ is Ala, Asn, or Gin; X₂₇ is Val or Leu; X₂₈ is Arg or Ala; X₂₉ is Gly or Gin; X₃₀ is Gly or Lys;
Y1 is a Lys, Orn, Dap, Dab, or Cys residue comprising a substituent on a side chain, the substituent having a formula {[2-(2-amino-ethoxy)-ethoxy]-acetyl}ₐ-(y-Glu)_{b}-CO-(CH₂)_{c}-COOH;
a is an integer of 1-3;
b is 1 or 2;
c is an integer of 10-30.

3. The pharmaceutical composition according to claim 1 or 2, wherein:
X₁ is Tyr; X₂ is Aib; X₁₀ is Tyr; X₁₂ is Ile; X₁₃ is Tyr; X₁₄ is Y1; X₁₅ is Asp or Glu; X₁₆ is Arg or Lys; X₁₇ is Ile; X₁₈ is Ala; X₁₉ is Ala; X₂₀ is Gin; X₂₃ is Ile or Val; X₂₄ is Asn; X₂₇ is Ile or Leu; X₂₈ is Ala; X₂₉ is Gly; X₃₀ is Gly;
Y1 is as defined in claim 2.

4. The pharmaceutical composition according to claim 3, wherein:
X₁₆ is Lys; X₂₃ is Val; X₂₇ is Leu.

5. The pharmaceutical composition according to claim 1, wherein:
X₁ is Tyr; X₂ is Aib or D-Ala; X₁₀ is Y1; X₁₂ is Ile; X₁₃ is Tyr; X₁₄ is Leu or Nle; X₁₅ is Glu; X₁₆ is Arg or Lys; X₁₇ is Ile; X₁₈ is Ala; X₁₉ is Ala; X₂₀ is Gln or Lys; X₂₃ is Ile or Val; X₂₄ is Asn or Gin; X₂₇ is Ile or Leu; X₂₈ is Ala; X₂₉ is Gly; X₃₀ is Gly;
Y1 is as defined in claim 2.

6. The pharmaceutical composition according to claim 1, wherein:
X₁ is Tyr; X₂ is Aib or D-Ala; X₁₀ is Tyr; X₁₂ is Y1; X₁₃ is Tyr; X₁₄ is Leu or Nle; X₁₅ is Glu; X₁₆ is Arg or Lys; X₁₇ is Ile; X₁₈ is Ala; X₁₉ is Ala; X₂₀ is Gln or Lys; X₂₃ is Ile or Val; X₂₄ is Asn or Gin; X₂₇ is Ile or Leu; X₂₈ is Ala; X₂₉ is Gly; X₃₀ is Gly;
Y1 is as defined in claim 2.

7. The pharmaceutical composition according to claim 1, wherein:
X₁ is Tyr; X₂ is Aib or D-Ala; X₁₀ is Tyr; X₁₂ is Ile; X₁₃ is Y1; X₁₄ is Leu or Nle; X₁₅ is Glu; X₁₆ is Arg or Lys; X₁₇ is Ile; X₁₈ is Ala; X₁₉ is Ala; X₂₀ is Gln or Lys; X₂₃ is Ile or Val; X₂₄ is Asn or Gin; X₂₇ is Ile or Leu; X₂₈ is Ala; X₂₉ is Gly; X₃₀ is Gly;
Y1 is as defined in claim 2.

8. The pharmaceutical composition according to claim 1, wherein:
X₁ is Tyr; X₂ is Aib or D-Ala; X₁₀ is Tyr; X₁₂ is Ile; X₁₃ is Tyr; X₁₄ is Y1; X₁₅ is Glu; X₁₆ is Arg or Lys; X₁₇ is Ile; X₁₈ is Ala; X₁₉ is Ala; X₂₀ is Gln or Lys; X₂₃ is Ile or Val; X₂₄ is Asn or Gin; X₂₇ is Ile or Leu; X₂₈ is Ala; X₂₉ is Gly; X₃₀ is Gly;
Y1 is as defined in claim 2.

9. The pharmaceutical composition according to claim 8, wherein:
X₂ is Aib; X₂₀ is Gin; X₂₄ is Asn.

10. The pharmaceutical composition according to claim 1, wherein:
X₁ is Tyr; X₂ is Aib or D-Ala; X₁₀ is Tyr; X₁₂ is Ile; X₁₃ is Tyr; X₁₄ is Leu or Nle; X₁₅ is Glu; X₁₆ is Y1; X₁₇ is Ile; X₁₈ is Ala; X₁₉ is Ala; X₂₀ is Gln or Lys; X₂₃ is Ile or Val; X₂₄ is Asn or Gin; X₂₇ is Ile or Leu; X₂₈ is Ala; X₂₉ is Gly; X₃₀ is Gly;
Y1 is as defined in claim 2.

11. The pharmaceutical composition according to claim 10, wherein:
X₂ is Aib; X₁₄ is Leu; X₂₀ is Gin; X₂₄ is Asn.

12. The pharmaceutical composition according to claim 1, wherein:
X₁ is Tyr; X₂ is Aib or D-Ala; X₁₀ is Tyr; X₁₂ is Ile; X₁₃ is Tyr; X₁₄ is Leu or Nle; X₁₅ is Glu; X₁₆ is Arg or Lys; X₁₇ is Y1; X₁₈ is Ala; X₁₉ is Ala; X₂₀ is Gln or Lys; X₂₃ is Ile or Val; X₂₄ is Asn or Gin; X₂₇ is Ile or Leu; X₂₈ is Ala; X₂₉ is Gly; X₃₀ is Gly;
Y1 is as defined in claim 2.

13. The pharmaceutical composition according to any one of claims 2-12, wherein a is 2, b is 1 or 2, and c is an integer of 16-20.

14. The pharmaceutical composition according to claim 13, wherein c is 16, 18, or 20.

15. The pharmaceutical composition according to any one of claims 2-14, wherein Y1 is a Lys residue comprising a substituent on a side chain, the substituent having a formula {[2-(2-amino-ethoxy)-ethoxy]-acetyl}ₐ-(y-Glu)_{b}-CO-(CH₂)_{c}-COOH;
a is 2;
b is 1 or 2;
c is 16 or 18.

16. The pharmaceutical composition according to any one of claims 2-15, wherein the substituent is covalently connected to amino on the side chain via an amide bond.

17. The pharmaceutical composition according to any one of claims 2-16, wherein Y1 is K(-OEG-OEG-yGlu-C18-OH) or K(-OEG-OEG-yGlu-C20-OH),
wherein K(-OEG-OEG-yGlu-C18-OH) has a structure shown below: 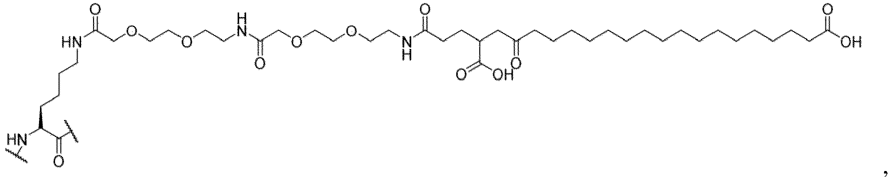 and
preferably has a structure shown below: 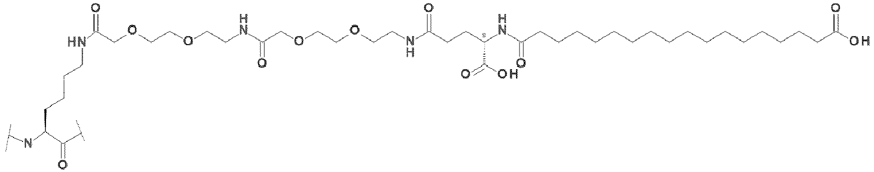
and K(-OEG-OEG-yGlu-C20-OH) has a structure shown below: 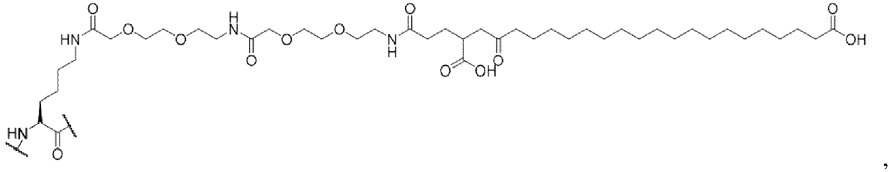
and preferably has a structure shown below:

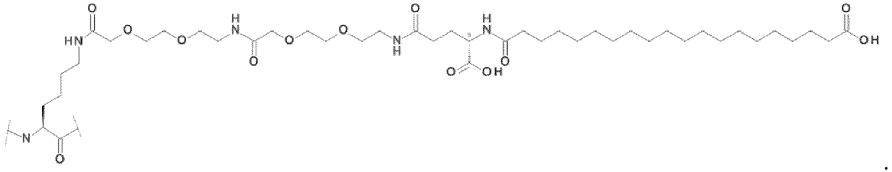

18. The pharmaceutical composition according to any one of claims 2-17, wherein the substituent is covalently connected to ε amino on the side chain via an amide bond.

19. The pharmaceutical composition according to claim 1, wherein the GLP-1 analog has the sequence set forth in SEQ ID NO: 20;
preferably, the GLP-1 analog is selected from any one of the following compounds numbered 1 to 18:
| | |
|---|---|
| 1 | H-YAibEGTFTSDYSIYKDKIAAQEFVNWLIAGGPSSGAPPPS-NH₂ |
| 2 | H-YAibEGTFTSDYSIYKDRIAAQEFVNWLIAGGPSSGAPPPS-NH₂ |
| 3 | H-YAibEGTFTSDYSIYKDKIAAQEFINWLIAGGPSSGAPPPS-NH₂ |
| 4 | H-YAibEGTFTSDYSIYKDRIAAQEFINWLIAGGPSSGAPPPS-NH₂ |
| 5 | H-YAibEGTFTSDYSIYKDKIAAQEFINWLLAGGPSSGAPPPS-NH₂ |
| 6 | H-YAibEGTFTSDYSIYKDRIAAQEFVNWLLAGGPSSGAPPPS-NH₂ |
| 7 | H-YAibEGTFTSDYSIYKDKIAAQEFVNWLLAGGPSSGAPPPS-NH₂ |
| 8 | H-YAibEGTFTSDYSIYLEKIAAQEFVNWLLAGGPSSGAPPPS-NH₂ |
| 9 | H-YAibEGTFTSDYSIYLEKIAAQEFVNWLIAGGPSSGAPPPS-NH₂ |
| 10 | H-YAibEGTFTSDYSIYLEKIAAQEFINWLIAGGPSSGAPPPS-NH₂ |
| 11 | H-YAibEGTFTSDYSIYLEKIAAQEFINWLLAGGPSSGAPPPS-NH₂ |
| 12 | H-YAibEGTFTSDYSIYKEKIAAQEFVNWLIAGGPSSGAPPPS-NH₂ |
| 13 | H-YAibEGTFTSDYSIYKERIAAQEFVNWLIAGGPSSGAPPPS-NH₂ |
| 14 | H-YAibEGTFTSDYSIYKEKIAAQEFINWLIAGGPSSGAPPPS-NH₂ |
| 15 | H-YAibEGTFTSDYSIYKERIAAQEFINWLIAGGPSSGAPPPS-NH₂ |
| 16 | H-YAibEGTFTSDYSIYKEKIAAQEFINWLLAGGPSSGAPPPS-NH₂ |
| 17 | H-YAibEGTFTSDYSIYKERIAAQEFVNWLLAGGPSSGAPPPS-NH₂ |
| 18 | H-YAibEGTFTSDYSIYKEKIAAQEFVNWLLAGGPSSGAPPPS-NH₂. |

20. The pharmaceutical composition according to claim 1, wherein the GLP-1 analog is selected from any one of the following compounds numbered 1# to 18#:
| | |
|---|---|
| 1# | H-YAibEGTFTSDYSIYK(OEG-OEG-yGlu-C20-OH)DKIAAQEFVNWLIAGGPSSGAPPPS-NH₂ |
| 2# | H-YAibEGTFTSDYSIYK(OEG-OEG-yGlu-C20-OH)DRIAAQEFVNWLIAGGPSSGAPPPS-NH₂ |
| 3# | H-YAibEGTFTSDYSIYK(OEG-OEG-yGlu-C20-OH)DKIAAQEFINWLIAGGPSSGAPPPS-NH₂ |
| 4# | H-YAibEGTFTSDYSIYK(OEG-OEG-yGlu-C20-OH)DRIAAQEFINWLIAGGPSSGAPPPS-NH₂ |
| 5# | H-YAibEGTFTSDYSIYK(OEG-OEG-yGlu-C20-OH)DKIAAQEFINWLLAGGPSSGAPPPS-NH₂ |
| 6# | H-YAibEGTFTSDYSIYK(OEG-OEG-yGlu-C20-OH)DRIAAQEFVNWLLAGGPSSGAPPPS-NH₂ |
| 7# | H-YAibEGTFTSDYSIYK(OEG-OEG-yGlu-C20-OH)DKIAAQEFVNWLLAGGPSSGAPPPS-NH₂ |
| 8# | H-YAibEGTFTSDYSIYLEK(OEG-OEG-yGlu-C20-OH)IAAQEFVNWLLAGGPSSGAPPPS-NH₂ |
| 9# | H-YAibEGTFTSDYSIYLEK(OEG-OEG-yGlu-C20-OH)IAAQEFVNWLIAGGPSSGAPPPS-NH₂ |
| 10# | H-YAibEGTFTSDYSIYLEK(OEG-OEG-yGlu-C20-OH)IAAQEFINWLIAGGPSSGAPPPS-NH₂ |
| 11# | H-YAibEGTFTSDYSIYLEK(OEG-OEG-yGlu-C20-OH)IAAQEFINWLLAGGPSSGAPPPS-NH₂ |
| 12# | H-YAibEGTFTSDYSIYK(OEG-OEG-yGlu-C20-OH)EKIAAQEFVNWLIAGGPSSGAPPPS-NH₂ |
| 13# | H-YAibEGTFTSDYSIYK(OEG-OEG-yGlu-C20-OH)ERIAAQEFVNWLIAGGPSSGAPPPS-NH₂ |
| 14# | H-YAibEGTFTSDYSIYK(OEG-OEG-yGlu-C20-OH)EKIAAQEFINWI,IAGGPSSGAPPPS-NH₂ |
| 15# | H-YAibEGTFTSDYSIYK(OEG-OEG-yGlu-C20-OH)ERIAAQEFINWI,IAGGPSSGAPPPS-NH₂ |
| 16# | H-YAibEGTFTSDYSIYK(OEG-OEG-yGlu-C20-OH)EKIAAQEFINWLLAGGPSSGAPPPS-NH₂ |
| 17# | H-YAibEGTFTSDYSIYK(OEG-OEG-yGlu-C20-OH)ERIAAQEFVNWLLAGGPSSGAPPPS-NH₂ |
| 18# | H-YAibEGTFTSDYSIYK(OEG-OEG-yGlu-C20-OH)EKIAAQEFVNWLLAGGPSSGAPPPS-NH₂. |

21. The pharmaceutical composition according to claim 1, wherein the GLP-1 analog is selected from the group consisting of compounds shown as 7#, 12#, 13#, 14#, 15#, 16#, 17#, and 18# in FIG. 3.

22. The pharmaceutical composition according to any one of claims 1-21, further comprising an osmotic pressure regulator, wherein
preferably, the osmotic pressure regulator is selected from one or more of propylene glycol, mannitol, sorbitol, xylitol, glycerol, lactose, trehalose, sucrose, glucose, sodium chloride, phosphate, sodium citrate, boric acid, and sodium tartrate;
more preferably, the osmotic pressure regulator is propylene glycol, sodium chloride, or mannitol;
most preferably, the osmotic pressure regulator is propylene glycol or sodium chloride.

23. The pharmaceutical composition according to any one of claims 1-22, further comprising a bacteriostatic agent, wherein
preferably, the bacteriostatic agent is selected from the group consisting of phenol, o-cresol, m-cresol, p-cresol, methyl paraben, propyl paraben, 2-phenoxyethanol, butyl paraben, 2-phenylethyl alcohol, benzyl alcohol, ethanol, chlorobutanol, thimerosal, bronopol, benzoic acid, imidurea, chlorhexidine, sodium dehydroacetate, chlorocresol, ethyl paraben, benzethonium chloride, and mixtures thereof, and
more preferably, the bacteriostatic agent is phenol.

24. The pharmaceutical composition according to any one of claims 1-23, further comprising a pH adjuster, wherein preferably, the pH adjuster is sodium hydroxide and/or hydrochloric acid.

25. The pharmaceutical composition according to any one of claims 1-24, wherein the pH of the pharmaceutical composition is 7.0 to 8.0, preferably 7.1 to 7.7, and most preferably about 7.5 or about 7.4.

26. The pharmaceutical composition according to any one of claims 1-25, wherein the concentration of the GLP-1 analog or the pharmaceutically acceptable salt thereof is 0.1 mg/mL to 500 mg/mL, preferably 1.0 mg/mL to 100 mg/mL, more preferably 1.0 mg/mL to 30.0 mg/mL, and most preferably 2.0 mg/mL to 10.0 mg/mL.

27. The pharmaceutical composition according to any one of claims 1-26, wherein the concentration of the buffer in the pharmaceutical composition is 1.0 mM to 35.0 mM, preferably 1.0 mM to 25.0 mM, and more preferably 2.0 mM to 10.0 mM.

28. The pharmaceutical composition according to any one of claims 22-27, wherein: the concentration of propylene glycol in the pharmaceutical composition is 10 mg/mL to 20 mg/mL, preferably 12 mg/mL to 16 mg/mL, and more preferably about 14 mg/mL; or the concentration of mannitol in the pharmaceutical composition is 30 mg/mL to 45 mg/mL, preferably 30 mg/mL to 40 mg/mL, and more preferably about 31.5 mg/mL; or the concentration of sodium chloride in the pharmaceutical composition is 2 mg/mL to 18 mg/mL, preferably 8 mg/mL to 10 mg/mL, and more preferably about 9 mg/mL.

29. The pharmaceutical composition according to any one of claims 23-28, wherein: the concentration of the bacteriostatic agent in the pharmaceutical composition is 4.0 mg/mL to 7.0 mg/mL, preferably 4.4 mg/mL to 6.8 mg/mL, more preferably 5.5 mg/mL to 6.6 mg/mL, and most preferably about 5.5 mg/mL.

30. A pharmaceutical composition, comprising:
a compound shown as 18# in FIG. 3 or a pharmaceutically acceptable salt thereof; sodium dihydrogen phosphate; and
propylene glycol, mannitol, or sodium chloride;
wherein optionally, the pharmaceutical composition further comprises phenol.

31. The pharmaceutical composition according to claim 30, comprising any one of A)-J), the pH of the pharmaceutical composition being 6.5 to 9.0, wherein:
A) 1.0 mg/mL to 100 mg/mL compound shown as 18# in FIG. 3 or the pharmaceutically acceptable salt thereof,
1.0 mM to 35.0 mM sodium dihydrogen phosphate, and
10 mg/mL to 20 mg/mL propylene glycol or 30 mg/mL to 40 mg/mL mannitol;
or the pharmaceutical composition comprises:
B) 1.0 mg/mL to 100 mg/mL compound shown as 18# in FIG. 3 or the pharmaceutically acceptable salt thereof,
1.0 mM to 35.0 mM sodium dihydrogen phosphate, and
2 mg/mL to 18 mg/mL sodium chloride;
or the pharmaceutical composition comprises:
C) 1.0 mg/mL to 100 mg/mL compound shown as 18# in FIG. 3 or the pharmaceutically acceptable salt thereof,
1.0 mM to 35.0 mM sodium dihydrogen phosphate;
10 mg/mL to 20 mg/mL propylene glycol, or 15 mg/mL to 45 mg/mL mannitol, or 2 mg/mL to 18 mg/mL sodium chloride; and
optionally, the pharmaceutical composition further comprises an antibacterial agent, such as 4.0 mg/mL to 7.0 mg/mL phenol;
D) 1.0 mg/mL to 30.0 mg/mL compound shown as 18# in FIG. 3 or the pharmaceutically acceptable salt thereof,
1.0 mM to 25.0 mM sodium dihydrogen phosphate,
11 mg/mL to 18 mg/mL propylene glycol, or 20 mg/mL to 40 mg/mL mannitol, or 3 mg/mL to 15 mg/mL sodium chloride, and
optionally, an antibacterial agent, such as 4.2 mg/mL to 6.9 mg/mL phenol;
the pH of the pharmaceutical composition is 7.0 to 8.0;
E) 2.0 mg/mL to 10.0 mg/mL compound shown as 18# in FIG. 3 or the pharmaceutically acceptable salt thereof,
2.0 mM to 10.0 mM sodium dihydrogen phosphate,
12 mg/mL to 16 mg/mL propylene glycol, or 25 mg/mL to 35 mg/mL mannitol, or 8 mg/mL to 10 mg/mL sodium chloride, and
optionally, an antibacterial agent, such as 4.4 mg/mL to 6.8 mg/mL phenol;
the pH of the pharmaceutical composition is 7.1 to 7.7;
F) 1.0 mg/mL to 100 mg/mL compound shown as 18# in FIG. 3 or the pharmaceutically acceptable salt thereof,
1.0 mM to 35.0 mM sodium dihydrogen phosphate,
10 mg/mL to 20 mg/mL propylene glycol, or 15 mg/mL to 45 mg/mL mannitol, or 2 mg/mL to 18 mg/mL sodium chloride,
optionally, an antibacterial agent, such as 4.0 mg/mL to 7.0 mg/mL phenol, and
water for injection;
the pH of the pharmaceutical composition is 6.5 to 9.0;
G) 1.0 mg/mL to 30.0 mg/mL compound shown as 18# in FIG. 3 or the pharmaceutically acceptable salt thereof,
1.0 mM to 25.0 mM sodium dihydrogen phosphate,
11 mg/mL to 18 mg/mL propylene glycol, or 20 mg/mL to 40 mg/mL mannitol, or 3 mg/mL to 15 mg/mL sodium chloride,
optionally, an antibacterial agent, such as 4.2 mg/mL to 6.9 mg/mL phenol, and
water for injection;
the pH of the pharmaceutical composition is 7.0 to 8.0;
H) 2.0 mg/mL to 20.0 mg/mL compound shown as 18# in FIG. 3 or the pharmaceutically acceptable salt thereof,
2.0 mM to 10.0 mM sodium dihydrogen phosphate,
12 mg/mL to 16 mg/mL propylene glycol, or 25 mg/mL to 35 mg/mL mannitol, or 7 mg/mL to 10 mg/mL sodium chloride,
optionally, an antibacterial agent, such as 4.4 mg/mL to 6.8 mg/mL phenol, and
water for injection;
the pH of the pharmaceutical composition is 7.1 to 7.7;
I) 5.0 mg/mL to 15.0 mg/mL or 5.0 mg/mL to 10.0 mg/mL compound shown as 18# in FIG. 3 or the pharmaceutically acceptable salt thereof,
5.0 mM to 10.0 mM sodium dihydrogen phosphate,
12 mg/mL to 16 mg/mL propylene glycol, or 7 mg/mL to 10 mg/mL sodium chloride, or
8 mg/mL to 9 mg/mL sodium chloride,
optionally, an antibacterial agent, such as 4.4 mg/mL to 6.8 mg/mL phenol, and
water for injection;
the pH of the pharmaceutical composition is 7.1 to 7.7;
J) 5.0 mg/mL to 15.0 mg/mL or 5.0 mg/mL to 10.0 mg/mL compound shown as 18# in FIG. 3 or the pharmaceutically acceptable salt thereof,
4.0 mM to 8.0 mM or 4.0 mM to 6.0 mM sodium dihydrogen phosphate,
12 mg/mL to 16 mg/mL propylene glycol, or 7 mg/mL to 10 mg/mL sodium chloride, or
8 mg/mL to 9 mg/mL sodium chloride,
optionally, an antibacterial agent, such as 4.4 mg/mL to 6.8 mg/mL phenol, and
water for injection;
the pH of the pharmaceutical composition is 7.1 to 7.7.

32. The pharmaceutical composition according to any one of claims 30-31, wherein:
(1) the pharmaceutical composition comprises about 2.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, and about 5.5 mg/mL phenol, and the pH of the pharmaceutical composition is about 7.5 or about 7.4;
(2) the pharmaceutical composition comprises about 4.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, and about 5.5 mg/mL phenol, and the pH of the pharmaceutical composition is about 7.5 or about 7.4;
(3) the pharmaceutical composition comprises about 5.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, and about 5.5 mg/mL phenol, and the pH of the pharmaceutical composition is about 7.5 or about 7.4;
(4) the pharmaceutical composition comprises about 6.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, and about 5.5 mg/mL phenol, and the pH of the pharmaceutical composition is about 7.5 or about 7.4;
(5) the pharmaceutical composition comprises about 8.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, and about 5.5 mg/mL phenol, and the pH of the pharmaceutical composition is about 7.5 or about 7.4;
(6) the pharmaceutical composition comprises about 10.0 mg/mL compound shown as 18# in FIG. 3 or the pharmaceutically acceptable salt thereof, about 5.0 mM sodium dihydrogen phosphate, about 14.0 mg/mL propylene glycol or about 31.5 mg/mL mannitol, and about 5.5 mg/mL phenol, and the pH of the pharmaceutical composition is about 7.5 or about 7.4;
or
(7) the pharmaceutical composition comprises about 2.0 mg/mL, about 4.0 mg/mL, about 5.0 mg/mL, about 6.0 mg/mL, about 8.0 mg/mL, 10 mg/mL, or about 20 mg/mL compound shown as 18# in FIG. 3 or the pharmaceutically acceptable salt thereof, about 5.0 mM sodium dihydrogen phosphate, about 9 mg/mL sodium chloride, and optionally about 5.5 mg/mL phenol, and the pH of the pharmaceutical composition is about 7.5 or about 7.4.

33. A method for preparing the pharmaceutical composition according to any one of claims 1-32, comprising the step of dissolving the GLP-1 analog or the pharmaceutically acceptable salt thereof.

34. A lyophilized formulation, wherein the lyophilized formulation is capable of forming the pharmaceutical composition according to any one of claims 1-32 upon reconstitution, or the lyophilized formulation is obtained by lyophilizing the pharmaceutical composition according to any one of claims 1-32.

35. A reconstituted solution, wherein the reconstituted solution is prepared by reconstituting the lyophilized formulation according to claim 17.

36. An article of manufacture, comprising a container, wherein the container contains the pharmaceutical composition according to any of claims 1-32, the lyophilized formulation according to claim 34, or the reconstituted solution according to claim 35.

37. Use of the pharmaceutical composition according to any one of claims 1-33, the lyophilized formulation according to claim 34, the reconstituted solution according to claim 35, or the article of manufacture according to claim 36 in the preparation of a medicament for treating non-insulin-dependent diabetes, insulin-dependent diabetes, obesity, non-alcoholic fatty liver, hepatic steatosis, diabetic retinopathy, diabetic neuropathy, diabetic nephropathy, insulin resistance, dyslipidemia associated with insulin resistance, and/or dyslipidemia associated with diabetes.
